(19) Europäisches Patentamt
European Patent Office
Office européen des brevets

(11) Numéro de publication: **0 485 251 B1**

(12) **FASCICULE DE BREVET EUROPEEN**

(45) Date de publication de fascicule du brevet: **01.03.95**

(51) Int. Cl.[6]: **A61K 9/127**, A61K 7/00, A23P 1/04

(21) Numéro de dépôt: **91402787.5**

(22) Date de dépôt: **18.10.91**

(54) **Composition cosmétique, pharmaceutique ou alimentaire comportant une dispersion de vésicules lipidiques.**

(30) Priorité: **09.11.90 FR 9013917**

(43) Date de publication de la demande:
**13.05.92 Bulletin 92/20**

(45) Mention de la délivrance du brevet:
**01.03.95 Bulletin 95/09**

(84) Etats contractants désignés:
**AT BE CH DE DK ES FR GB GR IT LI NL SE**

(56) Documents cités:
**EP-A- 0 100 382**
**EP-A- 0 180 980**
**WO-A-88/10147**
**GB-A- 2 177 092**

**DIE PHARMAZIE, vol. 45, no. 10, octobre 1990, pages 747-749, Berlin, DE; P.SCHENK et al.: "Studies on sucrose-palmitate-stearate-containing vesiclesencapsulating the cytostatic drug methylglyoxal-bis-guanyl-hydrazone"**

(73) Titulaire: **L'OREAL**
**14, Rue Royale**
**F-75008 Paris (FR)**

(72) Inventeur: **Morancais, Jean-Luc**
**11, rue Georges Brassens**
**F-77330 Ozoir-La-Ferriere (FR)**
Inventeur: **Philippe, Michel**
**3, rue de l'Aubépine**
**F-92160 Antony (FR)**

(74) Mandataire: **Peuscet, Jacques et al**
**SCP Cabinet Peuscet et Autres,**
**68 Rue d'Hauteville**
**F-75010 Paris (FR)**

**EP 0 485 251 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

**DERWENT FILE SUPPLIER WPI(L), 1986, AN=86-282499 [43], Derwent PublicationsLtd, Londres, GB; & JP-A-61 207 324 (MITSUBISHI CHEM. IND.) 13-09-1986**

**DERWENT FILE SUPPLIER WPI(L), 1989, AN=89-073938 [10], Derwent PublicationsLtd, Londres, GB; & JP-A-1 027 637 (DAIICHI SEIYAKU K.K.) 30-01-1989**

**CHEM. PHARM. BULL., vol. 33, no. 2, février 1985, pages 753-759, Tokyo, JP; H.KIWADA et al.: "Application of synthetic alkyl glycoside vesicles as drugcarriers. I. Preparation and physical properties"**

## Description

La présente invention est relative à une composition cosmétique, pharmaceutique ou alimentaire comportant une dispersion de vésicules lipidiques en milieu aqueux.

On sait que les vésicules lipidiques de ces dispersions ont une structure lamellaire constituée d'au moins deux feuillets lipidiques disposés concentriquement pour définir un volume fermé ; elles encapsulent une phase aqueuse comprenant avantageusement des substances actives hydrosolubles, par exemple pharmaceutiques, alimentaires ou cosmétiques, lesquelles sont ainsi protégées des conditions extérieures. Ces vésicules peuvent être réalisées à partir de lipides ioniques ou de lipides non-ioniques.

Différentes familles d'agents tensio-actifs sont déjà connues comme pouvant constituer des feuillets de vésicules lipidiques ; à titre d'exemples, on peut mentionner les phospholipides, les glycéroglycolipides, les éthers de polyglycérol et les composés ammonium-quaternaires tels que le bromure de didodécyldiméthyl-ammonium ; les éthers de glucose, comme les gluco-pyrannosides d'alkyle, par exemple d'hexadécyle, peuvent aussi permettre la formation de telles vésicules. On pourra, à cet égard, se reporter à l'article "Les Niosomes" G.VANLERBERGHE et coll., Colloques Nationaux du C.N.R.S. n° 938, Bordeaux - 1978 , ainsi qu'à la publication de H.KIWADA et Coll. Chem. Pharm. Bull., 33 (2) pages 753-759, 1985.

La Société déposante a maintenant découvert qu'une famille de dérivés O-acylés (à groupe acyle en $C_{10}$-$C_{18}$) en position 6 du glucose, très facilement biodégradables et présentant une cytotoxicité très faible, inférieure à celle des glucopyrannosides d'alkyle, est également capable de former les feuillets de vésicules lipidiques dans des dispersions aqueuses qui peuvent être utilisées dans les domaines cosmétique, pharmaceutique et alimentaire. Ces esters de glucose n'ont jamais été proposés pour cette application. Dans l'état de la technique, on a, certes, décrit l'utilisation d'esters d'acides gras et d'hexose, dont le groupe acyle contient de 7 à 10 atomes de carbone, pour constituer des compositions moussantes que l'on peut employer dans les domaines du nettoyage, de l'hygiène corporelle et de l'industrie alimentaire (demande internationale WO 88/10147). Il n'était cependant pas évident que des esters de glucose de ce type puissent permettre la formation de vésicules lipidiques.

La présente invention a donc d'abord pour objet une composition cosmétique. Pharmaceutique ou alimentaire comportant, en dispersion dans un milieu aqueux D, des vésicules lipidiques à structure lamellaire encapsulant une phase aqueuse E, caractérisée par le fait que la phase lipidique formant les feuillets desdites vésicules est au moins en partie constituée par au moins un composé de formule (I) :

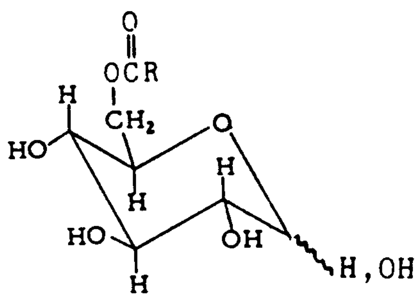

(I)

dans laquelle R représente une channe hydrocarbonée, linéaire, saturée ou insaturée, contenant de 9 à 17 atomes de carbone.

Les composés de formule (I) sont des composés connus,dont un procédé de préparation est décrit ci-après, et ils sont choisis,notamment,dans le groupe de ceux dont le reste acyle R-CO- est un reste décanoyle, dodécanoyle, myristoyle, hexadécanoyle, stéaroyle, oléoyle, linoléoyle ou linolénoyle. A titre d'exemples de composés de formule (I), on peut mentionner le O-oléoyl-6-D-glucose, le O-décanoyl-6-D-glucose, le O-dodécanoyl-6-D-glucose et le O-hexadécanoyl-6-D-glucose.Ces composés présentent l'avantage d'avoir une très faible cytotoxicité et d'être très facilement biodégradables avec libération du glucose et de l'acide RCOOH.

La phase lipidique formant les feuillets des vésicules peut renfermer également au moins un lipide amphiphile complémentaire, d'origine natuelle ou synthétique, ionique et/ou non-ionique, comportant, par molécule, au moins un groupement hydrophile pris dans le groupe formé par les groupements hydroxyle, étheroxyde, carboxyle, phosphate et amine.

Les lipides amphiphiles ioniques complémentaires peuvent être pris dans le groupe formé par :

- les phospholipides naturels, tels que la lécithine d'oeuf ou de soja et la sphingomyéline ;

- les phospholipides de synthèse, tels que la dipalmitoyl-phosphatidylcholine ou les lécithines hydrogénées ;
- les composés amphotères ; et
- les composés anioniques.

Les lipides amphiphiles non-ioniques complémentaires peuvent être pris dans le groupe formé par :

(1) les éthers de polyglycérol, linéaires ou ramifiés, de formule :

$$R^0O-[C_3H_5(OH)O]_{\overline{n}}-H$$

dans laquelle :

- $-C_3H_5(OH)O$ est représenté par les structures suivantes prises en mélange ou séparément : $-CH_2CHOHCH_2O-$ ;

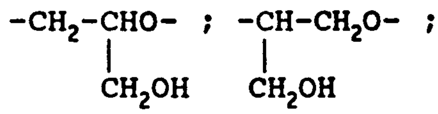

- $\overline{n}$ est une valeur statistique moyenne comprise entre 1 et 6 ;
- $R^0$ représente :
  (a) une chaîne aliphatique, linéaire ou ramifiée, saturée ou insaturée, contenant de 12 à 30 atomes de carbone ; ou des radicaux hydrocarbonés des alcools de lanoline : ou les restes des $\alpha$-diols à longue chaîne ;
  (b) un reste $R^1CO$, où $R^1$ est un radical aliphatique, linéaire ou ramifié, en $C_{11}$-$C_{17}$ ;
  (c) un reste $R^2[OC_2H_3(R^3)]$, où :
  . $R^2$ peut prendre la signification (a) ou (b) donnée pour $R^0$ ;
  . $OC_2H_3(R^3)$ - est représenté par les structures suivantes, prises enmélange ou séparément :

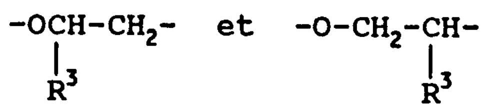

où $R^3$ prend la signification (a) donnée pour $R^0$ ;

(2) les éthers de polyglycérol, linéaires ou ramifiés, comportant deux channes grasses ;
(3) les alcools gras polyoxyéthylénés et les stérols et phytostérols polyoxyéthylénés ;
(4) les éthers de polyols ;
(5) les esters de polyols, oxyéthylénés ou non ;
(6) les glycolipides d'origine naturelle ou synthétiques ;
(7) les hydroxyamides représentés par la formule :

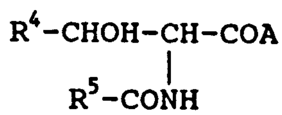

dans laquelle :

- $R^4$ désigne un radical alkyle ou alcényle en $C_7$-$C_{21}$ ;
- $R^5$ désigne un radical hydrocarboné, saturé ou insaturé, en $C_7$-$C_{31}$ ;
- COA désigne un groupement choisi parmi les deux groupements suivants :

- un reste

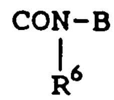

où :

. B est un radical dérivé d'amines primaires ou secondaires, mono- ou polyhydroxylées ; et

. $R^6$ désigne un atome d'hydrogène ou un radical méthyle, éthyle ou hydroxyéthyle ; et

- un reste -COOZ, où Z représente le reste d'un polyol en $C_3$-$C_7$.

Les vésicules lipidiques de la composition selon l'invention ont, de façon habituelle, un diamètre moyen généralement compris entre 20 et 5000 nm.

On peut préparer la composition selon l'invention par tous procédés connus, par exemple celui décrit dans le brevet français 2 315 991. Le procédé décrit dans ce brevet français n° 2 315 991 consiste à mettre en contact le (ou les) lipide(s) destiné(s) à constituer les feuillets des vésicules avec la phase aqueuse à encapsuler dans lesdites vésicules, les lipides étant capables de gonfler dans ladite phase aqueuse afin de former une phase lamellaire ; à agiter pour assurer le mélange et obtenir une phase lamellaire ; puis à ajouter un liquide de dispersion en une quantité supérieure à la quantité de phase lamellaire obtenue et à secouer énergiquement.

On peut également mettre en oeuvre le procédé décrit dans la demande de brevet européen n° 90-4026481, qui comprend la succession d'étapes consistant à dissoudre au moins un lipide dans au moins un solvant organique non miscible à l'eau ; ajouter la phase organique ainsi obtenue à une phase aqueuse ; former une dispersion des deux phases sous forte agitation, la taille des vésicules pouvant être réglée en faisant varier la vitesse d'agitation au cours de ce mélange des phases ; conduire l'évaporation du (ou des) solvant(s) sous forte agitation ; et, le cas échéant, concentrer la dispersion.

Par ailleurs, de façon connue, le(s) lipide(s) formant les vésicules peut (peuvent) être associé(s) à au moins un additif pris dans le groupe formé par :

- les alcools et diols à longue channe ;
- les stérols, par exemple, le cholestérol ;
- les amines à longue channe et leurs dérivés ammonium-quaternaires ;
- les hydroxyalkylamines, les dihydroxyalkylamines; les amines grasses polyoxyéthylénées, les esters d'amino-alcools à longue channe et leurs sels et dérivés ammonium-quaternaires ;
- les esters phosphoriques d'alcools gras, par exemple, le dicétylphosphate acide ou son sel de sodium ;
- les alkylsulfates, par exemple le cétylsulfate de sodium ;
- certains polymères, tels que les polypeptides et les protéines ; et
- des lipoprotides choisis parmi les dérivés mono- ou polyacylés d'amino-acides ou de polypeptides, dans lequel le reste acyle R'-CO- comporte une channe hydrocarbonée R'en $C_{13}$-$C_{19}$, au moins une des fonctions qui relie la channe polypeptidique ou le reste d'amino-acide à la channe lipophile étant une fonction amide, les fonctions carboxyliques de la channe polypeptidique ou du reste d'amino-acide pouvant être partiellement ou totalement neutralisées par un ou plusieurs cations alcalins, un ion ammonium ou un ammonium substitué dérivé d'une amine, ledit (ou lesdits) lipoprotide(s) étant présent(s) à un taux de 1 à 15% en poids par rapport au poids total de ladite phase lipidique propre. De tels lipoprotides sont décrits de façon détaillée dans le brevet français n° 2 597 345.

La composition selon l'invention contient avantageusement de 0,5 à 25% en poids de lipide(s) constituant les feuillets des vésicules, consistant en au moins un composé de formule (I), le cas échéant en mélange avec au moins un autre composé tel que décrit ci-dessus, ces pourcentages étant exprimés en poids par rapport au poids total de la composition.

Selon un mode de réalisation préféré, la phase aqueuse E, encapsulée dans-les vésicules, est une solution aqueuse de substance(s) active(s), de préférence sensiblement iso-osmotique par rapport à la phase D qui entoure les vésicules.

Pour une composition cosmétique selon l'invention, la phaseaqueuse encapsulée E et/ou la phase aqueuse de dispersion D peut(vent) renfermer au moins une substance cosmétique hydrosoluble prise dans le groupe formé par les humectants, les agents de brunissage artificiel, les agents de coloration de la peau, les agents anti-solaires, les filtres solaires, les agents anti-perspirants, les déodorants, les astringents, les produits rafraîchissants, les produits toniques, les produits cicatrisants, les produits kératolytiques, les produits dépilatoires, les eaux parfumées, les colorants hydrosolubles, les agents antipelliculaires, les

agents anti-séborrhéigues, les oxydants, les réducteurs et les extraits de tissus animaux ou végétaux.

Pour une composition pharmaceutique selon l'invention, la phase aqueuse E et/ou la phase aqueuse D peut (vent) également renfermer au moins un produit pris dans le groupe formé par les vitamines, les hormones, les enzymes, les vaccins, les anti-inflammatoires, les antibiotiques et les bactéricides.

Selon une variante de l'invention, au moins une phase liquide L, non miscible à l'eau, est dispersée dans la phase aqueuse D. En particulier la composition selon l'invention peut contenir de 2 à 70% en poids de phase liquide L, non miscible à l'eau, par rapport au poid total de la composition, la proportion pondérale relative de(s) lipide(s) constitutif(s) de vésicules par rapport à la phase liquide dispersée L étant comprise entre 0,02/1 à 10/1. En particulier, le(s) constituant(s) de la phase liquide L dispersée dans la phase aqueuse D,peut (peuvent) être choisi(s) dans le groupe formé par les huiles,telles que les esters d'acides gras et de polyols, et les esters d'acide gras et d'alcools ramifiés de formule $R^7$-$COOR^8$, formule dans laquelle $R^7$ représente le reste d'un acide gras,supérieur comportant de 7 à 19 atomes de carbone et $R^8$ représente une channe hydrocarbonée ramifiée contenant'de 3 à 20 atomes de carbone ; les hydrocarbures, tels que l'hexadécane, l'huile de paraffine, le perhydrosqualène ; les carbures halogénés,tels que le perfluorodécahydronaphtalène ; la perfluorotributylamine ; les polysiloxanes; les esters d'acides organiques, les éthers et polyéthers. La phase liquide L peut renfermer au moins un parfum et/ou au moins une substance active liposoluble. De telles substances liposolubles peuvent être constituées par les filtres solaires liposolubles, les substances destinées à améliorer l'état des peaux sèches ou séniles, les tocophérols, les vitamines E ou F, la vitamine A et ses esters, l'acite rétinoïque, les antioxydants, les acides gras essentiels, l'acide glycyrrhétinique, les agents kératolytiques et les caroténoïdes.

La phase aqueuse D peut également renfermer au moins'un adjuvant pris dans le groupe formé par les opacifiants, les gélifiants, les arômes, les parfums, les filtres anti-solaires et les colorants.

La présente invention concerne également une composition alimentaire caractérisée par le fait que le(s) lipide(s)de formule (I) et l'(les) éventuel(s) additif(s) formant les feuillets des vésicules est(sont) choisi(s) parmi les lipides comestibles ; que la phase aqueuse E et la phase aqueuse D renferment éventuellement au moins une vitamine hydrosoluble et que le(s) constituant(s) de la phase L, lorsqu'elle est présente, est (ou sont) choisi(s) parmi les huiles comestibles et renferme(nt) éventuellement au moins une vitamine liposoluble.

Pour mieux faire comprendre l'objet de la présente invention, on va en décrire maintenant, à titre d'exemples purement illustratifs et non limitatifs, plusieurs modes de mise en oeuvre. Les exemples 1 à 4 décrivent la préparation d'esters de glucose de formule (I) et les exemples 4 et 5, la préparation de compositions cosmétiques selon l'invention utilisant de tels esters pour constituer des vésicules lipidiques entrant dans lesdites compositions.

Le mode opératoire général de la préparation de dérivés O-acylés en position 6 du D-glucose est le suivant :

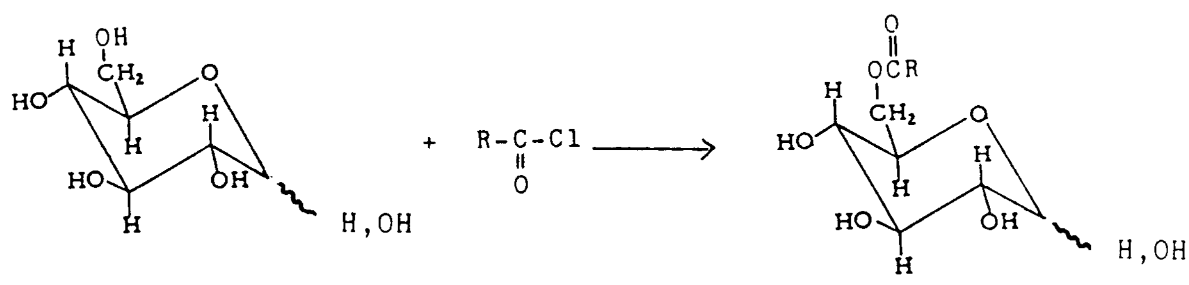

La synthèse est réalisée à partir du chlorure de l'acide choisi et du D-glucose selon la méthode décrite par E.REINEFELD et Coll., "Die Stärke", n° 6, pages 181-189, 1968 :

Dans un ballon de 2 litres, muni d'un dispositif d'agitation, d'un réfrigérant ascendant avec garde à chlorure de calcium, on introduit 72 g de D-glucose, 1 litre de pyridine, et on chauffe le mélange hétérogène à 70°C pendant 30 minutes jusqu'à obtention d'une solution liquide incolore. On refroidit le milieu réactionnel jusqu'à la température ambiante, et on y coule une solution du chlorure d'acide désiré (0,133 mole) dans 300 ml de tétrahydrofuranne. On agite le mélange résultant pendant 15 heures à la température ambiante en suivant l'évolution de la réaction en chromatographie sur couche mince (plaques de gel de silice "MERCK-5719" ; éluant :(méthanol/dichlorométhane, 15/85) ; révélateur : iode ).Puis le mélange est concentré à sec sous pression réduite. Le résidu est alors repris dans un mélange de-(dichlorométhane/méthanol, 93/7) puis chromatographié sur colonne de gel de silice (éluant : dichlorométhane/méthanol, 90/10), pour aboutir à l'isolement du O-acyl-6-D-glucopyrannose recherché (rendement :

25%).

On a étudié la cytotoxicité des O-acyl-6-D-glucopyrannoses ainsi obtenus en conduisant un essai in vitro d'inhibition de la croissance de cellules V 79 de fibroblastes pulmonaires de hamster chinois. Les cellules sont ensemencées à la densité de 5000 cellules par $cm^2$ dans des plaques de culture à 96 puits. Le milieu de culture contient 0,4 % en poids de diméthylsulfoxide. Après 24 heures d'incubation, les cellules sont traitées par des concentrations croissantes du glucopyrannose à étudier. La croissance cellulaire est appréciée après 3 jours de traitement par le dosage des protéines cellulaires totales. La concentration, exprimée en µg/ml, inhibitant la croissance'cellulaire de 50%, est déterminée ($CI_{50}$).

A des fins de comparaison, on a testé dans les mêmes conditions le glucopyrannoside 1 hexadécyle (non inclus dans les composés utilisables selon l'invention) ; la valeur trouvée pour la $CI_{50}$ de ce composé est de 12 µg/ml.

EXEMPLE 1 : Préparation du O-hexadécanoyl-6-D-glucose

En utilisant le chlorure d'hexadécanoyle, on a obtenu 23 g du produit de l'intitulé, dont le point de fusion est de 145 °C et qui est recristallisé dans l'acétonitrile.

L'analyse élémentaire du produit obtenu donne les résultats suivants :

| | C | H | O |
|---|---|---|---|
| Calculé pour $C_{22}H_{42}O_7$ (PM = 418,6) | 63,13 | 10,11 | 26,76 |
| Trouvé | 63,42 | 10,18 | |

Le spectre de résonance magnétique nucléaire du $^{13}C$ ($C_5D_5N$) est conforme à la structure attendue.

Dans l'étude de cytotoxicité, conduite comme indiqué ci-dessus, la limite de solubilité du produit obtenu n'a pas permis de le tester au-delà de 30 µg/ml, une valeur qui représente la $CI_{20}$, concentration à laquelle on observe environ 20% d'inhibition cellulaire.

EXEMPLE 2 : Préparation du O-oléoyl-6-D-glucose

En utilisant le chlorure d'oléoyle, on a obtenu 24 g du produit de l'intitulé, dont le point de fusion est de 114 °C et qui est recristallisé dans l'éther isopropylique.

L'analyse élémentaire du produit obtenu donne les résultats suivants :

| | C | H | O |
|---|---|---|---|
| Calculé pour $C_{24}H_{44}O_7$,$1H_2O$ (PM = 462,6) | 62,31 | 10,02 | 27,67 |
| Trouvé | 62,9 | 9,74 | |

Le spectre de résonance magnétique nucléaire du $^{13}C$ ($C_5D_5N$) est conforme à la structure attendue.

La concentration inhibitrice de croissance cellulaire $CI_{50}$ telle que définie ci-dessus, est trouvée égale à 59,3 µg/ml.

EXEMPLE 3 : Préparation du O-dodécanoyl-6-D-glucose

En utilisant le chlorure de dodécanoyle, on a obtenu 19,5 g du produit de l'intitulé, dont le point de fusion est de 121 °C et qui est recristallisé dans l'acétonitrile.

L'analyse élémentaire du produit obtenu donne les résultats suivants :

| | C | H | O |
|---|---|---|---|
| Calculé pour $C_{18}H_{34}O_7,2H_2O$ (PM = 398,5) | 54,25 | 9,61 | 36,13 |
| Trouvé | 53,72 | 9,28 | |

Le spectre de résonance magnétique nucléaire du $^{13}C$ ($C_5D_5N$) est conforme à la structure attendue.

La concentration inhibitrice de croissance cellulaire $CI_{50}$, telle que définie ci-dessus, est trouvée égale à 29,0 µg/ml.

EXEMPLE 4 : Préparation du O-décanoyl-6-D-glucose.

En utilisant le chlorure de décanoyle, on a obtenu 18 g du produit de l'intitulé, dont le point de fusion est de 124 °C et qui est recristallisé dans un mélange isopropanol/eau.

L'analyse élémentaire du produit obtenu donne les résultats suivants :

| | C | H | O |
|---|---|---|---|
| Calculé pour $C_{16}H_{30}O_7$ (PM = 334,4) | 57,47 | 9,04 | 33,49 |
| Trouvé | 57,36 | 9,08 | |

Le spectre de résonnance magnétique nucléaire du $^{13}C$ (diméthylsulfoxide deutérié) est conforme à la structure attendue.

EXAMPLE 5

On prépare une composition ayant la formulation suivante :

| | | |
|---|---|---|
| Composé de l'exemple 2 ............................ | 38 | g |
| Cholestérol ........................................ | 38 | g |
| Dicétylphosphate de Sodium ......................... | 4 | g |
| Conservateur ....................................... | 3 | g |
| Antioxydant ........................................ | 0,5 | g |
| Eau qsp ............................................ | 1000 | g |

Dans un mélange de 400 ml de dichlorométhane et de 400 ml de méthanol, on dissout 38 g de O-oleoyl-6-D-glucose, 38 g de cholestérol et 4 g de dicétylphosphate de sodium. La solution obtenue est évaporée sous pression réduite de façon progressive (d'environ 660 mbars à environ 13 mbars) dans un ballon rotatif porté à 40 °C.

Au film lipidique obtenu, on ajoute une solution aqueuse contenant 3 g de conservateur, 0,5 g d'antioxydant et 916,5 g d'eau. Le mélange est porté à 70 °C et agité pendant 2 h. à l'aide d'une secoueuse à bras oscillants.

La crème fluide ainsi obtenue est appliquée par voie topique à raison de 14 mg/cm$^2$ sur une zone de peau sèche, préalablement nettoyée, en une seule application.

L'application a été renouvelée quotidiennement. On a observé que cette crème non-grasse possédait de très bonnes qualités cosmétiques grâce à une grande facilité d'étalement et à une pénétration rapide au niveau de la peau.

EXEMPLE 6

On prépare une composition ayant la formulation suivante :

| | | |
|---|---|---|
| Composé de l'exemple 3 | 38 | g |
| Cholestérol | 38 | g |
| Dicétylphosphate de sodium | 4 | g |
| Conservateur | 3 | g |
| Anti-oxydant | 0,5 | g |
| Eau qsp | 1000 | g |

On opère exactement comme à l'exemple 5 ; la crème épaisse ainsi obtenue est appliquée par voie topique à raison de 15 mg/cm$^2$ sur une zone de peau sèche, préalablement nettoyée, en une seule application. L'application a été renouvelée quotidiennement. On a observé que cette crème non-grasse possédait de très bonnes qualités cosmétiques grâce à une grande facilité d'étalement et à une pénétration rapide au niveau de la peau.

Exemple 7

On prépare une composition ayant la formulation suivante :

| | | |
|---|---|---|
| - Composé de l'exemple 1 | 1,75 | g |
| - Cholestérol | 2,00 | g |
| - Dicétylphosphate de sodium | 0,25 | g |
| - Lécithine de soja vendue sous la dénomination "ASOLECTIN" par la Société FLUKA | 1,00 | g |
| - Glycérol | 1,90 | g |
| - Eau qsp | 100,00 | g |

Dans un mélange de 145 ml de dichlorométhane et de 115 ml de méthanol, on dissout 1,76 g de O-héxadécanoyl-6-D-glucose, 2 g de cholestérol, 0,25 g de dicétylphosphate de sodium et 1 g de lécithine de soja. La solution obtenue est ensuite évaporée sous pression réduite de façon progressive (d'environ 660 à environ 13 X 10$^2$ pascals) dans un ballon rotatif à 40 °C.
Au film lipidique obtenu, on ajoute une solution aqueuse contenant 1,9 g de glycérol et 93,1 g d'eau. Le milieu est alors désoxygéné par barbotage d'azote et dégazage en cuve à ultrasons.
Le mélange est porté à 45 °C et agité pendant 2 heures à l'aide d'une secoueuse à bras oscillants.
Après retour à la température ambiante, la dispersion obtenue est portée à la température de 30 °C puis ensuite traitée pendant 2 min à l'aide d'un homogénéisateur à ultrasons.
Le lait obtenu présente les mêmes qualités que la crème de l'exemple 5 et possède de plus un effet hydratant sur la peau.

Exemple 8

On prépare une composition ayant la formulation suivante :

| | |
|---|---|
| - Composé de l'exemple 1 ....... | 1,25 g |
| - Cholestérol .................. | 2,30 g |
| - Dicétylphosphate de sodium ... | 0,25 g |

- Lipide amphiphile non-ionique de formule :

$C_{16}H_{33}O-[C_3H_5(OH)O]_{\bar{n}}-H$

où $-C_3H_5(OH)O-$ est représenté par les structures suivantes, prises en mélange ou séparément

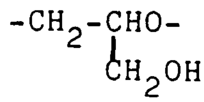

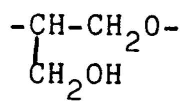

et $\bar{n}$ est une valeur statistique

| | |
|---|---|
| moyenne égale à 3 .............. | 1,13 g |
| - glycérol ..................... | 1,90 g |
| - eau ................ qsp | 100,00 g |

Dans un mélange de 16 ml de dichlorométhane et de 40 ml de méthanol, on dissout 1,25 g de O-hexadécanoyl 6-D-glucose, 2,3 g de cholestérol, 0,25 g de dicétylphosphate de sodium et 1,13 g du lipide amphiphile non-ionique décrit ci-dessus. La solution obtenue est ensuite évaporée sous pression réduite de façon progressive (d'environ 660 à environ 13 X $10^2$ pascals) dans un ballon rotatif à 40 ° C.

Au film lipidique obtenu, on ajoute une solution aqueuse contenant 1,9 g de glycérol et 93,1 g d'eau. Le milieu est alors désoxygéné par barbotage d'azote et dégazage en cuve à ultra-sons.

Le mélange est porté à 70 ° C et agité pendant 2 heures à l'aide d'une secoueuse à bras oscillants.

Après retour à la température ambiante, la dispersion obtenue est portée à la température de 30 ° C puis ensuite traitée pendant 2 min à l'aide d'un homogénéisateur à ultrasons.

Le lait obtenu présente les mêmes qualités que celui de l'exemple 7.

**Revendications**

**Revendications pour les Etats contractants suivants : AT, BE, CH, DE, DK, FR, GB, IT, LI, NL, SE**

**1.** Composition à usage cosmétique, pharmaceutique ou alimentaire, comportant, en dispersion dans un milieu aqueux D, des vésicules lipidiques à structure lamellaire encapsulant une phase aqueuse E; caractérisée par le fait que la phase lipidique formant les feuillets desdites vésicules est au moins en partie constituée par au moins un composé de formule (I) :

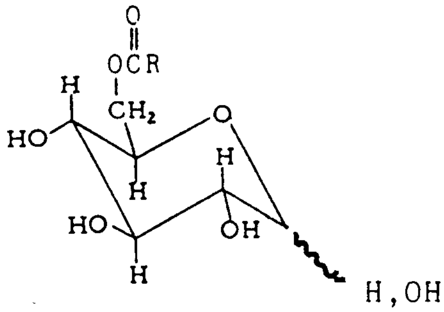

(I)

dans laquelle R représente une chaîne hydrocarbonée, linéaire, saturée ou insaturée, contenant de 9 à 17 atomes de carbone.

**2.** Composition selon la revendication 1, caractérisée par le fait que les composés de formule (I) sont choisis dans le groupe de ceux dont le reste acyle R-CO- est un reste décanoyle, dodécanoyle, myristoyle, hexadécanoyle, stéaroyle, oléoyle, linoléoyle ou linolénoyle.

**3.** Composition selon la revendication 2, caractérisée par le fait que les composés de formule (I) sont choisis dans le groupe formé par le O-oléoyl-6-D-glucose, le O-décanoyl-6-D-glucose, le O-dodécanoyl-6-D-glucose et le O-hexadécanoyl-6-D-glucose.

**4.** Composition selon l'une des revendications 1 à 3, caractérisée par le fait que la phase lipidique formant les feuillets des vésicules renferme au moins un lipide amphiphile complémentaire, d'origine naturelle ou synthétique, ionique et/ou non-ionique, comportant, par molécule, au moins un groupement hydrophile pris dans le groupe formé par les groupements hydroxyle, étheroxyde, carboxyle, phosphate et amine.

**5.** Composition selon la revendication 4, caractérisée par le fait que les lipides amphiphiles ioniques complémentaires sont pris dans le groupe formé par les phospholipides naturels ou de synthèse, les composés amphotères et les composés anioniques.

**6.** Composition selon la revendication 4, caractérisée par le fait que les lipides amphiphiles non-ioniques complémentaires sont pris dans le groupe formé par :

(1) les éthers de polyglycérol, linéaires ou ramifiés, de formule :

$$R^0O-[C_3H_5(OH)O]_{\bar{n}}-H$$

dans laquelle :

- $-C_3H_5(OH)O$ est représenté par les structures suivantes prises en mélange ou séparément : $-CH_2CHOHCH_2O-$ ;

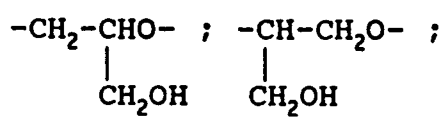

- $\bar{n}$ est une valeur statistique moyenne comprise entre 1 et 6 ;
- $R^0$ représente ;

  (a) une chaîne aliphatique, linéaire ou ramifiée, saturée ou insaturée, contenant de 12 à 30 atomes de carbone ; ou des radicaux hydrocarbonés des alcools de lanoline ; ou les restes des $\alpha$-diols à longue chaîne :

  (b) un reste $R^1CO$, où $R^1$ est un radical aliphatique, linéaire ou ramifié, en $C_{11}$-$C_{17}$ ;

  (c) un reste $R^2[OC_2H_3(R^3)]$, où :

  . $R^2$ peut prendre la signification (a) ou (b) donnée pour $R^0$ ;

. $OC_2H_3(R^3)$- est représenté par les structures suivantes, prises en mélange ou séparément :

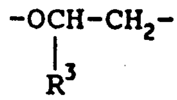 et 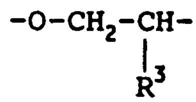

où $R^3$ prend la signification (a) donnée pour $R^0$ ;

(2) les éthers de polyglycérol, linéaires ou ramifiés, comportant deux chaînes grasses ;

(3) les alcools gras polyoxyéthylénés et les stérols et phytostérols polyoxyéthylénés ;

(4) les éthers de polyols ;

(5) les esters de polyols, oxyéthylénés ou non ;

(6) les glycolipides d'origine naturelle ou synthétiques ;

(7) les hydroxyamides représentés par la formule :

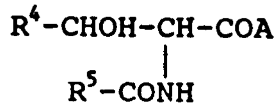

dans laquelle :

- $R^4$ désigne un radical alkyle ou alcényle en $C_7$-$C_{21}$ ;
- $R^5$ désigne un radical hydrocarboné, saturé ou insaturé, en $C_7$-$C_{31}$ ;
- COA désigne un groupement choisi parmi les deux groupements suivants :
  - un reste

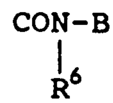

où :

. B est un radical dérivé d'amines primaires ou secondaires, mono- ou polyhydroxylées ; et

. $R^6$ désigne un atome d'hydrogène ou un radical méthyle, éthyle ou hydroxyéthyle ; et

- un reste -COOZ, où Z représente le reste d'un polyol en $C_3$-$C_7$.

**7.** Composition selon l'une des revendications 1 à 6, caractérisée par le fait que le(s) lipide(s) formant les vésicules est(sont) associé(s) à-au moins un additif pris dans le groupe formé par les alcools et diols à longue chaîne ; les stérols ; les amines à longue chaine et leurs dérivés ammonium-quaternaires ; les hydroxyalkylamines, les dihydroxyalkylamines, les amines grasses polyoxyéthylénées, les esters d'amino-alcools à longue chaîne, et leurs sels et dérivés ammonium-quaternaires ; les esters phosphoriques d'alcools gras ; les alkylsulfates ; des polymères de type polypeptides et protéines ; et des lipoprotides choisis parmi les dérivés mono- ou polyacylés d'amino-acides ou de polypeptides, dans lequel le reste acyle R'-CO- comporte une chaîne hydrocarbonée R' en $C_{13}$-$C_{19}$, au moins une des fonctions qui relie la chaîne polypeptidique ou le reste d'amino-acide à la chaîne lipophile étant une fonction amide, les fonctions carboxyliques de la chaîne polypeptidïque ou du reste d'amino-acide pouvant être partiellement ou totalement neutralisées par un ou plusieurs cations alcalins, un ion ammonium ou un ammonium substitué dérivé d'une amine.

**8.** Composition selon l'une des revendications 1 à 7, caractérisée par le fait qu'elle contient de 0,5 à 25% en poids de lipide(s) constituant les feuillets des vésicules, ces pourcentages étant exprimés en poids par rapport au poids total de la composition.

**9.** Composition selon l'une des revendications 1 à 8, caractérisée par le fait que la phase aqueuse E, encapsulée dans les vésicules, est une solution aqueuse de substance(s) active(s).

**10.** Composition cosmétique selon la revendication 9, caractérisée par le fait que la phase aqueuse E et/ou la phase aqueuse D renferme(nt) au moins une substance cosmétique hydrosoluble prise dans le groupe formé par les humectants, les agents de brunissage artificiel, les agents de coloration de la peau, les agents anti-solaires, les filtres solaires, les agents anti-perspirants, les déodorants, les astringents, les produits rafraîchissants, les produits toniques, les produits cicatrisants, les produits kératolytiques, les produits dépilatoires, les eaux parfumées, les colorants hydrosolubles, les agents anti-pelliculaires, les agents anti-séborrhéiques, les oxydants, les réducteurs et les extraits de tissus animaux ou végétaux.

**11.** Composition pharmaceutique selon la revendication 9, caractérisée par le fait que la phase aqueuse E et/ou la phase aqueuse D renferme(nt) au moins un produit pris dans le groupe formé par les vitamines, les hormones, les enzymes, les vaccins, les anti-inflammatoires, les antibiotiques et les bactéricides.

**12.** Composition selon l'une des revendications 1 à 11, caractérisée par le fait que la phase lipidique formant les feuillets des vésicules contient au moins une substance active liposoluble.

**13.** Composition selon la revendication 12, caractérisée par le fait que la substance active liposoluble est choisie dans le groupe formé par les filtres solaires liposolubles, les substances destinées à améliorer l'état des peaux sèches ou séniles, les tocophérols, les vitamines E ou F, la vitamine A et ses esters, l'acide rétinoïque, les antioxydants, les acides gras essentiels, l'acide glycyrrhétinique, les agents kératolytiques et les caroténoïdes.

**14.** Composition selon l'une des revendications 1 à 13, caractérisée par le fait qu'au moins une phase liquide L, non miscible à l'eau, est dispersée dans la phase aqueuse D.

**15.** Composition selon la revendication 14 caractérisée par le fait qu'elle contient de 2 à 70% en poids de phase liquide L, non miscible à l'eau, par rapport au poids total de la composition, la proportion pondérale relative de lipide(s) constitutif(s) de sphérules par rapport à la phase liquide dispersée L étant comprise entre 0,02/1 à 10/1.

**16.** Composition selon l'une des revendications 14 ou 15, caractérisée par le fait que le(s) constituant(s) de la phase liquide L, dispersée dans la phase aqueuse D, est (sont) choisi(s) dans le groupe constitué par les huiles telles que les esters d'acides gras et de polyols, et les esters d'acides gras et d'alcools ramifiés de formule $R^7$-$COOR^8$, formule dans laquelle $R^7$ représente le reste d'un acide gras supérieur comportant de 7 à 19 atomes de carbone et $R^8$ représente une chaîne hydrocarbonée ramifiée contenant de 3 à 20 atomes de carbone ; les hydrocarbures tels que l'hexadécane, l'huile de paraffine, le perhydrosqualène ; les carbures halogénés,tels que le perfluorodécahydronaphtalène ; la perfluorotributylamine ; les polysiloxanes ; les esters d'acides organiques, les éthers et polyéthers.

**17.** Composition selon l'une des revendications 14 à 16, caractérisée par le fait que la phase L renferme au moins un parfum et/ou au moins une substance active liposoluble.

**18.** Composition selon l'une des revendications 1 à 17 caractérisée par'le fait que la-phase aqueuse D renferme au moins un adjuvant pris dans le groupe formé par les opacifiants, les gélifiants, les arômes, les parfums, les filtres anti-solaires et les colorants.

**19.** Composition selon la revendication 18, caractérisée par le fait que la phase liposoluble est choisie dans le groupe formé par un filtre anti-solaire, une substance destinée à améliorer l'état des peaux sèches ou séniles ou un anti-oxydant.

**20.** Composition alimentaire selon l'une des revendications 1 à 9, 14 et 15, caractérisée par le fait que le(s) lipide(s) de formule (I) formant les feuillets des vésicules est(sont) choisi(s) parmi les lipides comestibles ; que la phase aqueuse E et la phase aqueuse D renferment éventuellement au moins une vitamine hydrosoluble et que le(s) constituant(s) de la phase L, lorsqu'elle est présente, est (sont) choisi(s) parmi les huiles comestibles et renferme(nt) éventuellement au moins une vitamine liposoluble.

**Revendications pour l'Etat contractant suivant : ES**

1. Composition à usage cosmétique, comportant, en dispersion dans un milieu aqueux D, des vésicules lipidiques à structure lamellaire encapsulant une phase aqueuse E, caractérisée par le fait que la phase lipidique formant les feuillets desdites vésicules est au moins en partie constituée par au moins un composé de formule (I) :

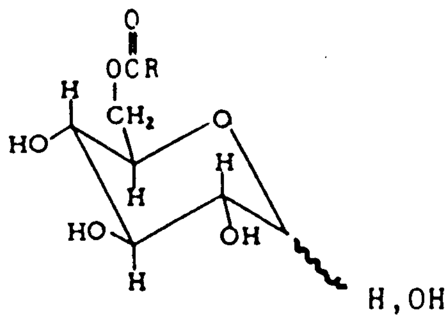

(I)

dans laquelle R représente une chaîne hydrocarbonée, linéaire, saturée ou insaturée, contenant de 9 à 17 atomes de carbone.

2. Composition selon la revendication 1, caractérisée par le fait que les composés de formule (I) sont choisis dans le groupe de ceux dont le reste acyle R-CO- est un reste décanoyle, dodécanoyle, myristoyle, hexadécanoyle, stéaroyle, oléoyle, linoléoyle ou linolénoyle.

3. Composition selon la revendication 2, caractérisée par le fait que les composés de formule (I) sont choisis dans le groupe formé par le O-oléoyl-6-D-glucose, le O-décanoyl-6-D-glucose, le O-dodécanoyl-6-D-glucose et le O-hexadécanoyl-6-D-glucose.

4. Composition selon l'une des revendications 1 à 3, caractérisée par le fait que la phase lipidique formant les feuillets des vésicules renferme au moins un lipide amphiphile complémentaire, d'origine naturelle ou synthétique, ionique et/ou non-ionique, comportant, par molécule, au moins un groupement hydrophile pris dans le groupe formé par les groupements hydroxyle, étheroxyde, carboxyle, phosphate et amine.

5. Composition selon la revendication 4, caractérisée par le fait que les lipides amphiphiles ioniques complémentaires sont pris dans le groupe formé par les phospholipides naturels ou de synthèse, les composés amphotères et les composés anioniques.

6. Composition selon la revendication 4, caractérisée par le fait que les lipides amphiphiles non-ioniques complémentaires sont pris dans le groupe formé par :
   (1) les éthers de polyglycérol, linéaires ou ramifiés, de formule :

$$R^0O-[C_3H_5(OH)O]_{\bar{n}}-H$$

   dans laquelle :

   - $-C_3H_5(OH)O$ est représenté par les structures suivantes prises en mélange ou séparément : $-CH_2CHOHCH_2O-$ ;

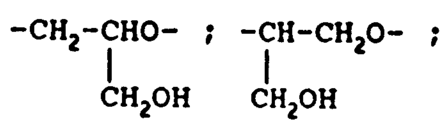

   - $\bar{n}$ est une valeur statistique moyenne comprise entre 1 et 6 ;

- $R^0$ représente :

  (a) une chaîne aliphatique, linéaire ou ramifiée, saturée ou insaturée, contenant de 12 à 30 atomes de carbone ; ou des radicaux hydrocarbonés des alcools de lanoline ; ou les restes des $\alpha$-diols à longue chaîne ;

  (b) un reste $R^1CO$, où $R^1$ est un radical aliphatique, linéaire ou ramifié, en $C_{11}$-$C_{17}$ ;

  (c) un reste $R^2$⫶$OC_2H_3(R^3)$⫶, où :

  . $R^2$ peut prendre la signification (a) ou (b) donnée pour $R^0$ ;

  . $OC_2H_3(R^3)$- est représenté par les structures suivantes, prises en mélange ou séparément :

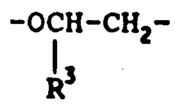

et

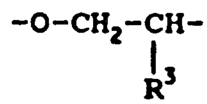

  où $R^3$ prend la signification (a) donnée pour $R^0$ ;

(2) les éthers de polyglycérol, linéaires ou ramifiés, comportant deux chaînes grasses ;
(3) les alcools gras polyoxyéthylénés et les stérols et phytostérols polyoxyéthylénés ;
(4) les éthers de polyols ;
(5) les esters de polyols, oxyéthylénés ou non ;
(6) les glycolipides d'origine naturelle ou synthétiques ;
(7) les hydroxyamides représentés par la formule :

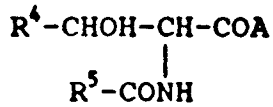

dans laquelle :

- $R^4$ désigne un radical alkyle ou alcényle en $C_7$-$C_{21}$ ;
- $R^5$ désigne un radical hydrocarboné, saturé ou insaturé, en $C_7$-$C_{31}$ ;
- COA désigne un groupement choisi parmi les deux groupements suivants :
  - un reste

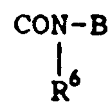

    où :

    . B est un radical dérivé d'amines primaires ou secondaires, mono- ou polyhydroxylées ; et

    . $R^6$ désigne un atome d'hydrogène ou un radical méthyle, éthyle ou hydroxyéthyle ; et
  - un reste -COOZ, où Z représente le reste d'un polyol en $C_3$-$C_7$.

**7.** Composition selon l'une des revendications 1 à 6, caractérisée par le fait que le(s) lipide(s) formant les vésicules est(sont) associé(s) à au moins un additif pris dans le groupe formé par les alcools et diols à longue chaîne ; les stérols ; les amines à longue chaîne et leurs dérivés ammonium-quaternaires ; les hydroxyalkylamines, les dihydroxyalkylamines, les amines grasses polyoxyéthylénées, les esters d'amino-alcools à longue chaîne, et leurs sels et dérivés ammonium-quaternaires ; les esters phosphoriques d'alcools gras ; les alkylsulfates ; des polymères de type polypeptides et protéines ; et des lipoprotides choisis parmi les dérivés mono- ou polyacylés d'amino-acides ou de polypeptides, dans lequel le reste acyle R'-CO- comporte une chaîne hydrocarbonée R'en $C_{13}$-$C_{19}$, au moins une des fonctions gui relie la chaîne polypeptidique ou le reste d'amino-acide à la chaîne lipophile étant une fonction amide, les fonctions carboxyliques de la chaîne polypeptidique ou du reste d'amino-acide pouvant être partiellement ou totalement neutralisées par un ou plusieurs cations alcalins, un ion ammonium ou un ammonium substitué dérivé d'une amine.

**8.** Composition selon l'une des revendications 1 à 7, caractérisée par le fait qu'elle contient de 0,5 à 25% en poids de lipide(s) constituant les feuillets des vésicules, ces pourcentages étant exprimés en poids par rapport au poids total de la composition.

**9.** Composition selon l'une des revendications 1 à 8, caractérisée par le fait que la phase aqueuse E, encapsulée dans les vésicules, est une solution aqueuse de substance(s) active(s).

**10.** Composition cosmétique selon la revendication 9, caractérisée par le fait que la phase aqueuse E et/ou la phase aqueuse D renferme(nt) au moins une substance cosmétique hydrosoluble prise dans le groupe formé par les humectants, les agents de brunissage artificiel, les agents de coloration de la peau, les agents anti-solaires, les filtres solaires, les agents anti-perspirants, les déodorants, les astringents, les produits rafraîchissants, les produits toniques, les produits cicatrisants, les produits kératolytiques, les produits dépilatoires, les eaux parfumées, les colorants hydrosolubles, les agents anti-pelliculaires, les agents anti-séborrhéiques, les oxydants, les réducteurs et les extraits de tissus animaux ou végétaux.

**11.** Composition selon l'une des revendications 1 à 10, caractérisée par le fait que la phase lipidique formant les feuillets des vésicules contient au moins une substance active liposoluble.

**12.** Composition selon la revendication 11 caractérisée par le fait que la substance active liposoluble est choisie dans le groupe formé par les filtres solaires liposolubles, les substances destinées à améliorer l'état des peaux sèches ou séniles, les tocophérols, les vitamines E ou F, la vitamine A et ses esters, l'acide rétinoïque, les antioxydants, les acides gras essentiels, l'acide glycyrrhétinique, les agents kératolytiques et les caroténoïdes.

**13.** Composition selon l'une des revendications 1 à 12, caractérisée par le fait qu'au moins une phase liquide L, non miscible à l'eau, est dispersée dans la phase aqueuse D.

**14.** Composition selon la revendication 13, caractérisée par le fait qu'elle contient de 2 à 70% en poids de phase liquide L, non miscible à l'eau, par rapport au poids total de la composition, la proportion pondérale relative de lipide(s) constitutif(s) de sphérules par rapport à la phase liquide dispersée L étant comprise entre 0,02/1 à 10/1.

**15.** Composition selon l'une des revendications 13 ou 14 , caractérisée par le fait que le(s) constituant(s) de la phase liquide L, dispersée dans la phase aqueuse D, est(sont) choisi(s) dans le groupe constitué par les huiles telles que les esters d'acides gras et de polyols, et les esters d'acides gras et d'alcools ramifiés de formule $R^7$-$COOR^8$, formule dans laquelle $R^7$ représente le reste d'un acide gras supérieur comportant de 7 à 19 atomes de carbone et $R^8$ représente une chaîne hydrocarbonée ramifiée contenant de 3 à 20 atomes de carbone ; les hydrocarbures tels que l'hexadécane, l'huile de paraffine, le perhydrosqualène ; les carbures halogénés,tels que le perfluorodécahydronaphtalène ; la perfluorotributylamine ; les polysiloxanes ; les esters d'acides organiques, les éthers et polyéthers.

**16.** Composition selon l'une des revendications 13 à 15, caractérisée par le fait que la phase L renferme au moins un parfum et/ou au moins une substance active liposoluble.

**17.** Composition selon l'une des revendications 1 à 16, caractérisée par le fait que la phase aqueuse D renferme au moins un adjuvant pris dans le groupe formé par les opacifiants, les gélifiants, les arômes, les parfums, les filtres anti-solaires et les colorants.

**18.** Composition selon la revendication 17, caractérisée par le fait que la phase liposoluble est choisie dans le groupe formé par un filtre anti-solaire, une substance destinée à améliorer l'état des peaux sèches ou séniles ou un anti-oxydant.

**19.** Procédé de fabrication d'une composition à usage cosmétique, pharmaceutique ou alimentaire, caractérisé par le fait qu'on prépare une dispersion dans un milieu aqueux D de vésicules lipidiques à structure lamellaire encapsulant une phase aqueuse E à partir d'une phase lipidique qui est au moins en partie constituée par au moins un composé de formule I

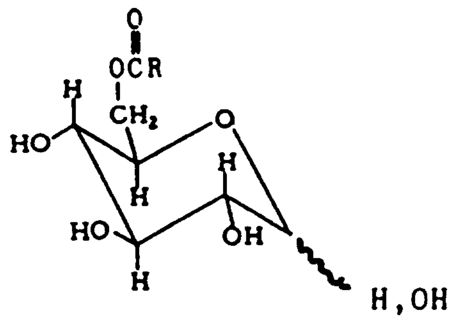

(I)

dans laquelle R représente une chaîne hydrocarbonée linéaire, saturée ou insaturée, contenant 9 à 17 atomes de carbone.

**Revendications pour l'Etat contractant suivant : GR**

1. Composition à usage cosmétique ou alimentaire,comportant, en dispersion dans un milieu aqueux D, des vésicules lipidiques à structure lamellaire encapsulant une phase aqueuse E, caractérisée par le fait que la phase lipidique formant les feuillets desdites vésicules est au moins en partie constituée par au moins un composé de formule (I) :

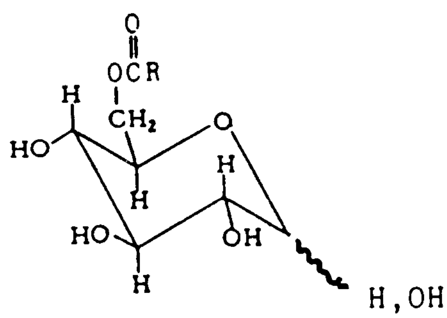

(I)

dans laquelle R représente une chaîne hydrocarbonée, linéaire, saturée ou insaturée, contenant de 9 à 17 atomes de carbone.

2. Composition selon la revendication 1, caractérisée par le fait que les composés de formule (I) sont choisis dans le groupe de ceux dont le reste acyle R-CO- est un reste décanoyle, dodécanoyle, myristoyle, hexadécanoyle, stéaroyle, oléoyle, linoléoyle ou linolénoyle.

3. Composition selon la revendication 2, caractérisée par le fait que les composés de formule (I) sont choisis dans le groupe formé par le O-oléoyl-6-D-glucose, le O-décanoyl-6-D-glucose, le O-dodécanoyl-6-D-glucose et le O-hexadécanoyl-6-D-glucose.

4. Composition selon l'une des revendications 1 à 3, caractérisée par le fait que la phase lipidique formant les feuillets des vésicules renferme au moins un lipide amphiphile complémentaire, d'origine naturelle ou synthétique, ionique et/ou non-ionique, comportant, par molécule, au moins un groupement hydrophile pris dans le groupe formé par les groupements hydroxyle, étheroxyde, carboxyle, phosphate et amine.

5. Composition selon la revendication 4, caractérisée par le fait que les lipides amphiphiles ioniques complémentaires sont pris dans le groupe formé par les phospholipides naturels ou de synthèse, les composés amphotères et les composés anioniques.

6. Composition selon la revendication 4, caractérisée par le fait que les lipides amphiphiles non-ioniques complémentaires sont pris dans le groupe formé par :

(1) les éthers de polyglycérol, linéaires ou ramifiés, de formule :

$$R^0O-[C_3H_5(OH)O]_{\overline{n}}-H$$

dans laquelle :

- $-C_3H_5(OH)O$ est représenté par les structures suivantes prises en mélange ou séparément : $-CH_2CHOHCH_2O-$ ;

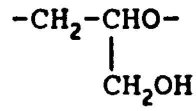 ; 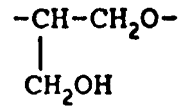 ;

- $\overline{n}$ est une valeur statistique moyenne comprise entre 1 et 6 ;
- $R^0$ représente :

  (a) une chaîne aliphatique, linéaire ou ramifiée, saturée ou insaturée, contenant de 12 à 30 atomes de carbone ; ou des radicaux hydrocarbonés des alcools de lanoline ; ou les restes des $\alpha$-diols à longue chaîne ;

  (b) un reste $R^1CO$, où $R^1$ est un radical aliphatique, linéaire ou ramifié, en $C_{11}$-$C_{17}$ ;

  (c) un reste $R^2[OC_2H_3(R^3)]$, où :

  . $R^2$ peut prendre la signification (a) ou (b) donnée pour $R^0$ ;

  . $OC_2H_3(R^3)$- est représenté par les structures suivantes, prises en mélange ou séparément :

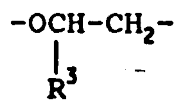 et 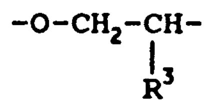

où $R^3$ prend la signification (a) donnée pour $R^0$ ;

(2) les éthers de polyglycérol, linéaires ou ramifiés, comportant deux chaînes grasses ;

(3) les alcools gras polyoxyéthylénés et les stérols et phytostérols polyoxyéthylénés ;

(4) les éthers de polyols ;

(5) les esters de polyols, oxyéthylénés ou non ;

(6) les glycolipides d'origine naturelle ou synthétiques ;

(7) les hydroxyamides représentés par la formule :

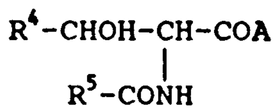

dans laquelle :

- $R^4$ désigne un radical alkyle ou alcényle en $C_7$-$C_{21}$ ;
- $R^5$ désigne un radical hydrocarboné, saturé ou insaturé, en $C_7$-$C_{31}$ ;
- COA désigne un groupement choisi parmi les deux groupements suivants :
  - un reste

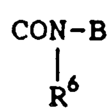

où :

. B est un radical dérivé d'amines primaires ou secondaires, mono- ou polyhydroxylées ; et

. $R^6$ désigne un atome d'hydrogène ou un radical méthyle, éthyle ou hydroxyéthyle ; et

- un reste -COOZ, où Z représente le reste d'un polyol en $C_3$-$C_7$.

**7.** Composition selon l'une des revendications 1 à 6, caractérisée par le fait que le(s) lipide(s) formant les vésicules est(sont) associé(s) à au moins un additif pris dans le groupe formé par les alcools et diols à longue chaîne ; les stérols ; les amines à longue chaîne et leurs dérivés ammonium-quaternaires ; les hydroxyalkylamines, les dihydroxyalkylamines, les amines grasses polyoxyéthylénées, les esters d'amino-alcools à longue chaîne, et leurs sels et dérivés ammonium-quaternaires ; les esters phosphoriques d'alcools gras ; les alkylsulfates ; des polymères de type polypeptides et protéines ; et des lipoprotides choisis parmi les dérivés mono- ou polyacylés d'amino-acides ou de polypeptides, dans lequel le reste acyle R'-CO- comporte une chaîne hydrocarbonée R'en $C_{13}$-$C_{19}$, au moins une des fonctions qui relie la chaîne polypeptidique ou le reste d'amino-acide à la chaîne lipophile étant une fonction amide, les fonctions carboxyliques de la chaîne polypeptidique ou du reste d'amino-acide pouvant être partiellement ou totalement neutralisées par un ou plusieurs cations alcalins, un ion ammonium ou un ammonium substitué dérivé d'une amine.

**8.** Composition selon l'une des revendications 1 à 7, caractérisée par le fait qu'elle contient de 0,5 à 25% en poids de lipide(s) constituant les feuillets des vésicules, ces pourcentages étant exprimés en poids par rapport au poids total de la composition.

**9.** Composition selon l'une des revendications 1 à 8, caractérisée par le fait que la phase aqueuse E, encapsulée dans les vésicules, est une solution aqueuse de substance(s) active(s).

**10.** Composition cosmétique selon la revendication 9, caractérisée par le fait que la phase aqueuse E et/ou la phase aqueuse D renferme(nt) au moins une substance cosmétique hydrosoluble prise dans le groupe formé par les humectants, les agents de brunissage artificiel, les agents de coloration de la peau, les agents anti-solaires , les filtres solaires, les agents anti-perspirants, les déodorants, les astringents, les produits rafraîchissants, les produits toniques, les produits cicatrisants, les produits kératolytiques, les produits dépilatoires, les eaux parfumées, les colorants hydrosolubles, les agents anti-pelliculaires, les agents anti-séborrhéiques, les oxydants, les réducteurs et les extraits de tissus animaux ou végétaux.

**11.** Composition selon l'une des revendications 1 à 10, caractérisée par le fait que la phase lipidique formant les feuillets des vésicules contient au moins une substance active liposoluble.

**12.** Composition selon la revendication 11 caractérisée par le fait que la substance active liposoluble est choisie dans le groupe formé par les filtres solaires liposolubles, les substances destinées à améliorer l'état des peaux sèches ou séniles, les tocophérols, les vitamines E ou F, la vitamine A et ses esters, l'acide rétinoïque, les antioxydants, les acides gras essentiels, l'acide glycyrrhétinique, les agents kératolytiques et les caroténoïdes.

**13.** Composition selon l'une des revendications 1 à 12, caractérisée par le fait qu'au moins une phase liquide L, non miscible à l'eau, est dispersée dans la phase aqueuse D.

**14.** Composition selon la revendication 13, caractérisée par le fait qu'elle contient de 2 à 70% en poids de phase liquide L, non miscible à l'eau, par rapport au poids total de la composition, la proportion pondérale relative de lipide(s) constitutif(s) de sphérules par rapport à la phase liquide dispersée L étant comprise entre 0,02/1 à 10/1.

**15.** Composition selon l'une des revendications 13 ou 14, caractérisée par le fait que le(s) constituant(s) de la phase liquide L, dispersée dans la phase aqueuse D, est(sont) choisi(s) dans le groupe constitué par les huiles telles que les esters d'acides gras et de polyols, et les esters d'acides gras et d'alcools ramifiés de formule $R^7$-COOR$^8$, formule dans laquelle $R^7$ représente le reste d'un acide gras supérieur comportant de 7 à 19 atomes de carbone et $R^8$ représente une chaîne hydrocarbonée ramifiée contenant de 3 à 20 atomes de carbone ; les hydrocarbures tels que l'hexadécane, l'huile de paraffine, le perhydrosqualène ; les carbures halogénés,tels que le perfluorodécahydronaphtalène ; la perfluorotributylamine ; les polysiloxanes ; les esters d'acides organiques, les éthers et polyéthers.

**16.** Composition selon l'une des revendications 13 à 15, caractérisée par le fait que la phase L renferme au moins un parfum et/ou au moins une substance active liposoluble.

**17.** Composition selon l'une des revendications 1 à 16, caractérisée par le fait que la phase aqueuse D renferme au moins un adjuvant pris dans le groupe formé par les opacifiants, les gélifiants, les arômes, les parfums, les filtres anti-solaires et les colorants.

**18.** Composition selon la revendication 17, caractérisée par le fait que la phase liposoluble est choisie dans le groupe formé par un filtre anti-solaire, une substance destinée à améliorer l'état des peaux sèches ou séniles ou un anti-oxydant.

**19.** Composition alimentaire selon l'une des revendications 1 à 9, 13 et 14, caractérisée par le fait que le(s) lipide(s) de formule (I) formant les feuillets des vésicules est(sont) choisi(s) parmi les lipides comestibles ; que la phase aqueuse E et la phase aqueuse D renferment éventuellement au moins une vitamine hydrosoluble et que le(s) constituant(s) de la phase L, lorsqu'elle est présente, est (sont) choisi(s) parmi les huiles comestibles et renferme(nt) éventuellement au moins une vitamine liposoluble.

**20.** Procédé de fabrication d'une composition à usage cosmétique, pharmaceutique ou alimentaire, caractérisé par le fait qu'on prépare une dispersion dans un milieu aqueux D de vésicules lipidiques à structure lamellaire encapsulant une phase aqueuse E à partir d'une phase lipidique qui est au moins en partie constituée par au moins un composé de formule I

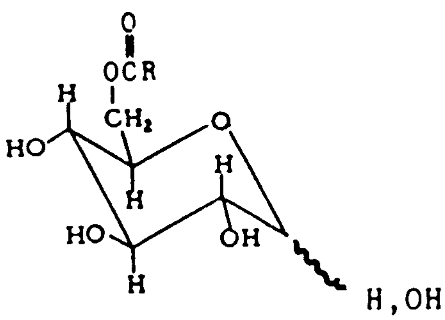

(I)

dans laquelle R représente une chaîne hydrocarbonée linéaire, saturée ou insaturée, contenant 9 à 17 atomes de carbone.

**Claims**

**Claims for the following Contracting States : AT, BE, CH, DE, DK, FR, GB, IT, LI, NL, SE**

**1.** Composition for cosmetic, pharmaceutical or food use, containing, in dispersion in an aqueous medium D, lipid vesicles of lamellar structure encapsulating an aqueous phase E, characterized by the fact that the lipid phase forming the sheets of the said vesicles at least partially consists of at least one compound of formula (I):

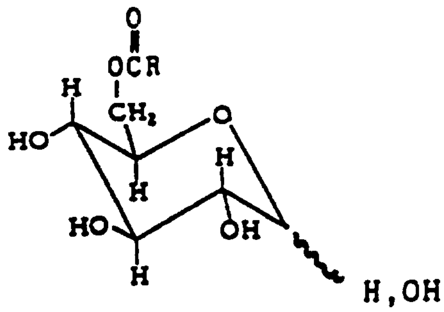

(I)

in which R represents a saturated or unsaturated linear hydrocarbon chain containing 9 to 17 carbon atoms.

2. Composition according to Claim 1, characterized by the fact that the compounds of formula (I) are chosen from the group comprising those whose acyl residue R-CO-is a decanoyl, dodecanoyl, myristoyl, hexadecanoyl, stearoyl, oleoyl, linoleoyl or linolenoyl residue.

3. Composition according to Claim 2, characterized by the fact that the compounds of formula (I) are chosen from the group consisting of O-oleoyl-6-D-glucose, O-decanoyl-6-D-glucose, O-dodecanoyl-6-D-glucose and O-hexadecanoyl-6-D-glucose.

4. Composition according to one of Claims 1 to 3, characterized by the fact that the lipid phase forming the sheets of the vesicles contains at least one additional amphiphilic lipid of natural or synthetic origin which is ionic and/or non-ionic, containing, per molecule, at least one hydrophilic group chosen from the group consisting of hydroxyl, ether, carboxyl, phosphate and amine groups.

5. Composition according to Claim 4, characterized by the fact that the additional ionic amphiphilic lipids are chosen from the group consisting of natural or synthetic phospholipids, amphoteric compounds and anionic compounds.

6. Composition according to Claim 4, characterized by the fact that the additional non-ionic amphiphilic lipids are chosen from the group consisting of:
(1) linear or branched polyglycerol ethers of formula:

$$R^0O-[C_3H_5(OH)O]_{\bar{n}}-H$$

in which:
. $-C_3H_5(OH)O$ is represented by the following structures taken as a mixture or separately: $-CH_2CHOHCH_2O-$;

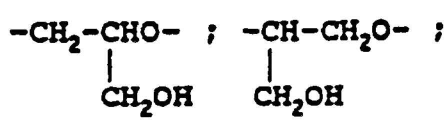

. $\bar{n}$ is a mean statistical value between 1 and 6;
. $R^0$ represents:
(a) a saturated or unsaturated, linear or branched, aliphatic chain containing 12 to 30 carbon atoms, or hydrocarbon radicals of lanolin alcohols, or the residues of long-chain α-diols;
(b) a residue $R^1CO$, where $R^1$ is a linear or branched $C_{11}$-$C_{17}$ aliphatic radical;
(c) a residue $R^2[OC_2H_3(R^3)]$, where:
. $R^2$ can have the meaning (a) or (b) which is given for $R^0$;

. $OC_2H_3(R^3)$- is represented by the following structures, taken as a mixture or separately:

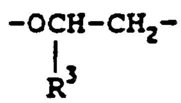

and

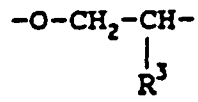

where $R^3$ has the meaning (a) which is given for $R^0$;

(2) linear or branched polyglycerol ethers containing two fatty chains;

(3) polyoxyethylenated fatty alcohols and polyoxyethylenated sterols and phytosterols;

(4) polyol ethers;

(5) oxyethylenated or non-oxyethylenated polyol esters;

(6) glycolipids of natural origin or which are synthetic;

(7) hydroxyamides represented by the formula:

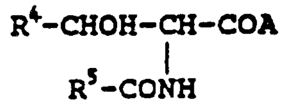

in which:

- $R^4$ designates a $C_7$-$C_{21}$ alkyl or alkenyl radical;
- $R^5$ designates a saturated or unsaturated $C_7$-$C_{31}$ hydrocarbon radical;
- COA designates a group chosen from the following two groups:
  . a residue

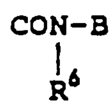

where:

. B is a radical derived from mono- or polyhydroxylated primary or secondary amines; and

. $R^6$ designates a hydrogen atom or a methyl, ethyl or hydroxyethyl radical; and

. a residue -COOZ, where Z represents the residue of a $C_3$-$C_7$ polyol.

**7.** Composition according to one of Claims 1 to 6, characterized by the fact that the lipid(s) constituting the vesicles is (are) combined with at least one additive chosen from the group consisting of long-chain alcohols and diols; sterols; long-chain amines and their quaternary ammonium derivatives; hydroxyalkylamines, dihydroxyalkylamines, polyoxyethylenated fatty amines, esters of long-chain amino alcohols, and their quaternary ammonium salts and derivatives; phosphoric esters of fatty alcohols; alkyl sulphates; polypeptide and protein type polymers; and lipoproteins chosen from mono- or polyacylated derivatives of amino acids or polypeptides, in which the acyl residue R'-CO- contains a $C_{13}$-$C_{19}$ hydrocarbon chain R', at least one of the functional groups which links the polypeptide chain or the amino acid residue to the lipophilic chain being an amide functional group, it being possible for the carboxylic functional groups of the polypeptide chain or of the amino acid residue to be partially or completely neutralized by one or more alkali metal cations, an ammonium ion or a substituted ammonium derived from an amine.

**8.** Composition according to one of Claims 1 to 7, characterized by the fact that it contains 0.5 to 25% by weight of lipid(s) constituting the vesicle sheets, these percentages being expressed by weight relative to the total weight of the composition.

**9.** Composition according to one of Claims 1 to 8, characterized by the fact that the aqueous phase E, which is encapsulated in the vesicles, is an aqueous solution of active substance(s).

10. Cosmetic composition according to Claim 9, characterized by the fact that the aqueous phase E and/or the aqueous phase D contain(s) at least one water-soluble cosmetic substance chosen from the group consisting of humectants, artificial tanning agents, skin colouring agents, anti-sun agents, sunscreens, antiperspirant agents, deodorants, astringents, refreshing products, tonic products, cicatrizing products, keratolytic products, depilatory products, perfumed water, water-soluble colorants, anti-dandruff agents, anti-seborrhoeic agents, oxidants, reducing agents and animal or plant tissue extracts.

11. Pharmaceutical composition according to Claim 9, characterized by the fact that the aqueous phase E and/or the aqueous phase D contain(s) at least one product chosen from the group consisting of vitamins, hormones, enzymes, vaccines, anti-inflammatory agents, antibiotics and bactericides.

12. Composition according to one of Claims 1 to 11, characterized by the fact that the lipid phase constituting the vesicle sheets contains at least one fat-soluble active substance.

13. Composition according to Claim 12, characterized by the fact that the fat-soluble active substance is chosen from the group consisting of fat-soluble sun-screens, substances intended to improve the condition of dry or aged skins, tocopherols, vitamins E or F, vitamin A and its esters, retinoic acid, antioxidants, essential fatty acids, glycyrrhetinic acid, keratolytic agents and carotenoids.

14. Composition according to one of Claims 1 to 13, characterized by the fact that at least one water-immiscible liquid phase L is dispersed in the aqueous phase D.

15. Composition according to Claim 14, characterized by the fact that it contains 2 to 70% by weight of water-immiscible liquid phase L relative to the total weight of the composition, the relative proportion by weight of lipid(s) constituting spherules relative to the dispersed liquid phase L being between 0.02/1 to 10/1.

16. Composition according to either of Claims 14 and 15, characterized by the fact that the constituent(s) of the liquid phase L, which is dispersed in the aqueous phase D, is (are) chosen from the group consisting of oils such as esters of fatty acids and polyols, and esters of fatty acids and branched alcohols of formula $R^7$-$COOR^8$, in which formula $R^7$ represents the residue of a higher fatty acid containing 7 to 19 carbon atoms and $R^8$ represents a branched hydrocarbon chain containing 3 to 20 carbon atoms; hydrocarbons such as hexadecane, paraffin oil, perhydrosqualene; halogenated hydrocarbons such as perfluorodecahydronaphthalene; perfluorotributylamine; polysiloxanes; esters of organic acids, ethers and polyethers.

17. Composition according to one of Claims 14 to 16, characterized by the fact that the phase L contains at least one perfume and/or at least one fat-soluble active substance.

18. Composition according to one of Claims 1 to 17, characterized by the fact that the aqueous phase D contains at least one adjuvant chosen from the group consisting of opacifiers, gelling agents, flavourings, perfumes, sunscreens and colorants.

19. Composition according to Claim 18, characterized by the fact that the fat-soluble phase is chosen from the group consisting of a sunscreen, a substance intended to improve the condition of dry or aged skins or an anti-oxidant.

20. Food composition according to one of Claims 1 to 9, 14 and 15, characterized by the fact that the lipid(s) of formula (I) constituting the vesicle sheets is (are) chosen from edible lipids; that the aqueous phase E and the aqueous phase D optionally contain at least one water-soluble vitamin and that the constituent(s) of the phase L, when it is present, is (are) chosen from edible oils and optionally contain(s) at least one fat-soluble vitamin.

**Claims for the following Contracting State : ES**

1. Composition for cosmetic use, containing, in dispersion in an aqueous medium D, lipid vesicles of lamellar structure encapsulating an aqueous phase E, characterized by the fact that the lipid phase forming the sheets of the said vesicles at least partially consists of at least one compound of formula

(I):

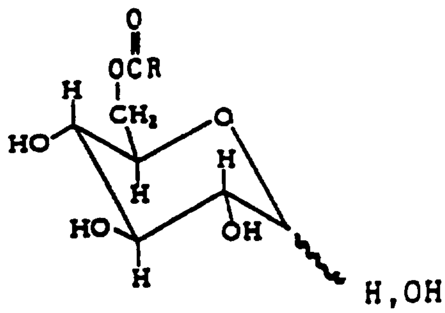

(I)

in which R represents a saturated or unsaturated linear hydrocarbon chain containing 9 to 17 carbon atoms.

**2.** Composition according to Claim 1, characterized by the fact that the compounds of formula (I) are chosen from the group comprising those whose acyl residue R-CO-is a decanoyl, dodecanoyl, myristoyl, hexadecanoyl, stearoyl, oleoyl, linoleoyl or linolenoyl residue.

**3.** Composition according to Claim 2, characterized by the fact that the compounds of formula (I) are chosen from the group consisting of O-oleoyl-6-D-glucose, O-decanoyl-6-D-glucose, O-dodecanoyl-6-D-glucose and O-hexadecanoyl-6-D-glucose.

**4.** Composition according to one of Claims 1 to 3, characterized by the fact that the lipid phase forming the sheets of the vesicles contains at least one additional amphiphilic lipid of natural or synthetic origin which is ionic and/or non-ionic, containing, per molecule, at least one hydrophilic group chosen from the group consisting of hydroxyl, ether, carboxyl, phosphate and amine groups.

**5.** Composition according to Claim 4, characterized by the fact that the additional ionic amphiphilic lipids are chosen from the group consisting of natural or synthetic phospholipids, amphoteric compounds and anionic compounds.

**6.** Composition according to Claim 4, characterized by the fact that the additional non-ionic amphiphilic lipids are chosen from the group consisting of:
(1) linear or branched polyglycerol ethers of formula:

$$R^0O-\!\!\left[C_3H_5(OH)O\right]_{\bar{n}}\!-H$$

in which:

. $-C_3H_5(OH)O$ is represented by the following structures taken as a mixture or separately:
$-CH_2CHOHCH_2O-$;

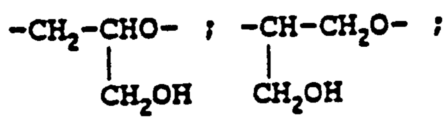

. $\bar{n}$ is a mean statistical value between 1 and 6;
. $R^0$ represents:
(a) a saturated or unsaturated, linear or branched, aliphatic chain containing 12 to 30 carbon atoms, or hydrocarbon radicals of lanolin alcohols, or the residues of long-chain $\alpha$-diols;
(b) a residue $R^1CO$, where $R^1$ is a linear or branched $C_{11}$-$C_{17}$ aliphatic radical;
(c) a residue $R^2\{OC_2H_3(R^3)\}$, where:

. $R^2$ can have the meaning (a) or (b) which is given for $R^0$;

. $OC_2H_3(R^3)$- is represented by the following structures, taken as a mixture or separately:

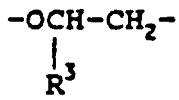 and 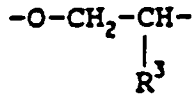

where $R^3$ has the meaning (a) which is given for $R^0$;

(2) linear or branched polyglycerol ethers containing two fatty chains;

(3) polyoxyethylenated fatty alcohols and polyoxyethylenated sterols and phytosterols;

(4) polyol ethers;

(5) oxyethylenated or non-oxyethylenated polyol esters;

(6) glycolipids of natural origin or which are synthetic;

(7) hydroxyamides represented by the formula:

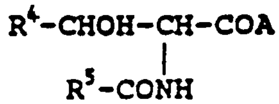

in which:

- $R^4$ designates a $C_7$-$C_{21}$ alkyl or alkenyl radical;
- $R^5$ designates a saturated or unsaturated $C_7$-$C_{31}$ hydrocarbon radical;
- COA designates a group chosen from the following two groups:

. a residue

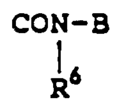

where:

. B is a radical derived from mono- or polyhydroxylated primary or secondary amines; and

. $R^6$ designates a hydrogen atom or a methyl, ethyl or hydroxyethyl radical; and

. a residue -COOZ, where Z represents the residue of a $C_3$-$C_7$ polyol.

**7.** Composition according to one of Claims 1 to 6, characterized by the fact that the lipid(s) constituting the vesicles is (are) combined with at least one additive chosen from the group consisting of long-chain alcohols and diols; sterols; long-chain amines and their quaternary ammonium derivatives; hydroxyalkylamines, dihydroxyalkylamines, polyoxyethylenated fatty amines, esters of long-chain amino alcohols, and their quaternary ammonium salts and derivatives; phosphoric esters of fatty alcohols; alkyl sulphates; polypeptide and protein type polymers; and lipoproteins chosen from mono- or polyacylated derivatives of amino acids or polypeptides, in which the acyl residue R'-CO- contains a $C_{13}$-$C_{19}$ hydrocarbon chain R', at least one of the functional groups which links the polypeptide chain or the amino acid residue to the lipophilic chain being an amide functional group, it being possible for the carboxylic functional groups of the polypeptide chain or of the amino acid residue to be partially or completely neutralized by one or more alkali metal cations, an ammonium ion or a substituted ammonium derived from an amine.

**8.** Composition according to one of Claims 1 to 7, characterized by the fact that it contains 0.5 to 25% by weight of lipid(s) constituting the vesicle sheets, these percentages being expressed by weight relative to the total weight of the composition.

**9.** Composition according to one of Claims 1 to 8, characterized by the fact that the aqueous phase E, which is encapsulated in the vesicles, is an aqueous solution of active substance(s).

**10.** Cosmetic composition according to Claim 9, characterized by the fact that the aqueous phase E and/or the aqueous phase D contain(s) at least one water-soluble cosmetic substance chosen from the group consisting of humectants, artificial tanning agents, skin colouring agents, anti-sun agents, sunscreens, antiperspirant agents, deodorants, astringents, refreshing products, tonic products, cicatrizing products, keratolytic products, depilatory products, perfumed water, water-soluble colorants, anti-dandruff agents, anti-seborrhoeic agents, oxidants, reducing agents and animal or plant tissue extracts.

**11.** Composition according to one of Claims 1 to 10, characterized by the fact that the lipid phase constituting the vesicle sheets contains at least one fat-soluble active substance.

**12.** Composition according to Claim 11, characterized by the fact that the fat-soluble active substance is chosen from the group consisting of fat-soluble sun-screens, substances intended to improve the condition of dry or aged skins, tocopherols, vitamins E or F, vitamin A and its esters, retinoic acid, antioxidants, essential fatty acids, glycyrrhetinic acid, keratolytic agents and carotenoids.

**13.** Composition according to one of Claims 1 to 12, characterized by the fact that at least one water-immiscible liquid phase L is dispersed in the aqueous phase D.

**14.** Composition according to Claim 13, characterized by the fact that it contains 2 to 70% by weight of water-immiscible liquid phase L relative to the total weight of the composition, the relative proportion by weight of lipid(s) constituting spherules relative to the dispersed liquid phase L being between 0.02/1 to 10/1.

**15.** Composition according to either of Claims 13 and 14, characterized by the fact that the constituent(s) of the liquid phase L, which is dispersed in the aqueous phase D, is (are) chosen from the group consisting of oils such as esters of fatty acids and polyols, and esters of fatty acids and branched alcohols of formula $R^7$-$COOR^8$, in which formula $R^7$ represents the residue of a higher fatty acid containing 7 to 19 carbon atoms and $R^8$ represents a branched hydrocarbon chain containing 3 to 20 carbon atoms; hydrocarbons such as hexadecane, paraffin oil, perhydrosqualene; halogenated hydrocarbons such as perfluorodecahydronaphthalene; perfluorotributylamine; polysiloxanes; esters of organic acids, ethers and polyethers.

**16.** Composition according to one of Claims 13 to 15, characterized by the fact that the phase L contains at least one perfume and/or at least one fat-soluble active substance.

**17.** Composition according to one of Claims 1 to 16, characterized by the fact that the aqueous phase D contains at least one adjuvant chosen from the group consisting of opacifiers, gelling agents, flavourings, perfumes, sunscreens and colorants.

**18.** Composition according to Claim 17, characterized by the fact that the fat-soluble phase is chosen from the group consisting of a sunscreen, a substance intended to improve the condition of dry or aged skins or an anti-oxidant.

**19.** Process for the manufacture of a composition for cosmetic, pharmaceutical or food use, characterized by the fact that a dispersion in an aqueous medium D of lipid vesicles of lamellar structure encapsulating an aqueous phase E is prepared from a lipid phase which at least partially consists of at least one compound of formula I

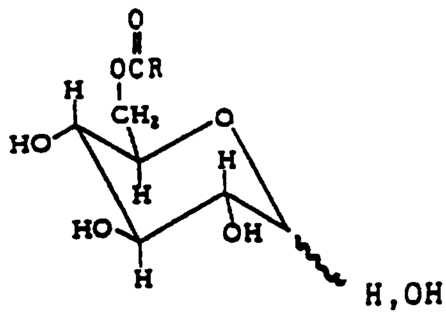

(I)

in which R represents a saturated or unsaturated linear hydrocarbon chain containing 9 to 17 carbon atoms.

**Claims for the following Contracting State : GR**

1. Composition for cosmetic or food use, containing, in dispersion in an aqueous medium D, lipid vesicles of lamellar structure encapsulating an aqueous phase E, characterized by the fact that the lipid phase forming the sheets of the said vesicles at least partially consists of at least one compound of formula (I):

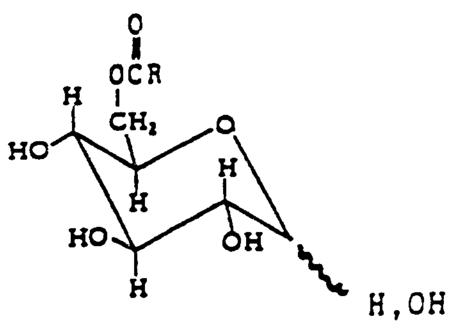

(I)

in which R represents a saturated or unsaturated linear hydrocarbon chain containing 9 to 17 carbon atoms.

2. Composition according to Claim 1, characterized by the fact that the compounds of formula (I) are chosen from the group comprising those whose acyl residue R-CO-is a decanoyl, dodecanoyl, myristoyl, hexadecanoyl, stearoyl, oleoyl, linoleoyl or linolenoyl residue.

3. Composition according to Claim 2, characterized by the fact that the compounds of formula (I) are chosen from the group consisting of O-oleoyl-6-D-glucose, O-decanoyl-6-D-glucose, O-dodecanoyl-6-D-glucose and O-hexadecanoyl-6-D-glucose.

4. Composition according to one of Claims 1 to 3, characterized by the fact that the lipid phase forming the sheets of the vesicles contains at least one additional amphiphilic lipid of natural or synthetic origin which is ionic and/or non-ionic, containing, per molecule, at least one hydrophilic group chosen from the group consisting of hydroxyl, ether, carboxyl, phosphate and amine groups.

5. Composition according to Claim 4, characterized by the fact that the additional ionic amphiphilic lipids are chosen from the group consisting of natural or synthetic phospholipids, amphoteric compounds and anionic compounds.

6. Composition according to Claim 4, characterized by the fact that the additional non-ionic amphiphilic lipids are chosen from the group consisting of:

(1) linear or branched polyglycerol ethers of formula:

$$R^0O-[C_3H_5(OH)O]_{\bar{n}}-H$$

in which:

. $-C_3H_5(OH)O$ is represented by the following structures taken as a mixture or separately: $-CH_2CHOHCH_2O-$;

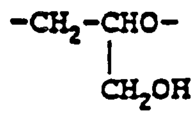 ; 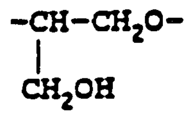 ;

. $\bar{n}$ is a mean statistical value between 1 and 6;

. $R^0$ represents:

(a) a saturated or unsaturated, linear or branched, aliphatic chain containing 12 to 30 carbon atoms, or hydrocarbon radicals of lanolin alcohols, or the residues of long-chain $\alpha$-diols;

(b) a residue $R^1CO$, where $R^1$ is a linear or branched $C_{11}$-$C_{17}$ aliphatic radical;

(c) a residue $R^2[OC_2H_3(R^3)]$, where:

. $R^2$ can have the meaning (a) or (b) which is given for $R^0$;

. $OC_2H_3(R^3)$- is represented by the following structures, taken as a mixture or separately:

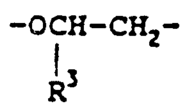 and 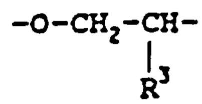

where $R^3$ has the meaning (a) which is given for $R^0$;

(2) linear or branched polyglycerol ethers containing two fatty chains;

(3) polyoxyethylenated fatty alcohols and polyoxyethylenated sterols and phytosterols;

(4) polyol ethers;

(5) oxyethylenated or non-oxyethylenated polyol esters;

(6) glycolipids of natural origin or which are synthetic;

(7) hydroxyamides represented by the formula:

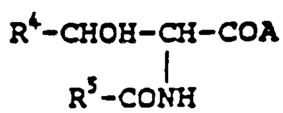

in which:

- $R^4$ designates a $C_7$-$C_{21}$ alkyl or alkenyl radical;
- $R^5$ designates a saturated or unsaturated $C_7$-$C_{31}$ hydrocarbon radical;
- COA designates a group chosen from the following two groups:

  . a residue

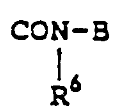

where:

. B is a radical derived from mono- or polyhydroxylated primary or secondary amines; and

. $R^6$ designates a hydrogen atom or a methyl, ethyl or hydroxyethyl radical; and
. a residue -COOZ, where Z represents the residue of a $C_3$-$C_7$ polyol.

**7.** Composition according to one of Claims 1 to 6, characterized by the fact that the lipid(s) constituting the vesicles is (are) combined with at least one additive chosen from the group consisting of long-chain alcohols and diols; sterols; long-chain amines and their quaternary ammonium derivatives; hydroxyalkylamines, dihydroxyalkylamines, polyoxyethylenated fatty amines, esters of long-chain amino alcohols, and their quaternary ammonium salts and derivatives; phosphoric esters of fatty alcohols; alkyl sulphates; polypeptide and protein type polymers; and lipoproteins chosen from mono- or polyacylated derivatives of amino acids or polypeptides, in which the acyl residue R'-CO- contains a $C_{13}$-$C_{19}$ hydrocarbon chain R', at least one of the functional groups which links the polypeptide chain or the amino acid residue to the lipophilic chain being an amide functional group, it being possible for the carboxylic functional groups of the polypeptide chain or of the amino acid residue to be partially or completely neutralized by one or more alkali metal cations, an ammonium ion or a substituted ammonium derived from an amine.

**8.** Composition according to one of Claims 1 to 7, characterized by the fact that it contains 0.5 to 25% by weight of lipid(s) constituting the vesicle sheets, these percentages being expressed by weight relative to the total weight of the composition.

**9.** Composition according to one of Claims 1 to 8, characterized by the fact that the aqueous phase E, which is encapsulated in the vesicles, is an aqueous solution of active substance(s).

**10.** Cosmetic composition according to Claim 9, characterized by the fact that the aqueous phase E and/or the aqueous phase D contain(s) at least one water-soluble cosmetic substance chosen from the group consisting of humectants, artificial tanning agents, skin colouring agents, anti-sun agents, sunscreens, antiperspirant agents, deodorants, astringents, refreshing products, tonic products, cicatrizing products, keratolytic products, depilatory products, perfumed water, water-soluble colorants, anti-dandruff agents, anti-seborrhoeic agents, oxidants, reducing agents and animal or plant tissue extracts.

**11.** Composition according to one of Claims 1 to 10, characterized by the fact that the lipid phase constituting the vesicle sheets contains at least one fat-soluble active substance.

**12.** Composition according to Claim 11, characterized by the fact that the fat-soluble active substance is chosen from the group consisting of fat-soluble sun-screens, substances intended to improve the condition of dry or aged skins, tocopherols, vitamins E or F, vitamin A and its esters, retinoic acid, antioxidants, essential fatty acids, glycyrrhetinic acid, keratolytic agents and carotenoids.

**13.** Composition according to one of Claims 1 to 12, characterized by the fact that at least one water-immiscible liquid phase L is dispersed in the aqueous phase D.

**14.** Composition according to Claim 13, characterized by the fact that it contains 2 to 70% by weight of water-immiscible liquid phase L relative to the total weight of the composition, the relative proportion by weight of lipid(s) constituting spherules relative to the dispersed liquid phase L being between 0.02/1 to 10/1.

**15.** Composition according to either of Claims 13 and 14, characterized by the fact that the constituent(s) of the liquid phase L, which is dispersed in the aqueous phase D, is (are) chosen from the group consisting of oils such as esters of fatty acids and polyols, and esters of fatty acids and branched alcohols of formula $R^7$-$COOR^8$, in which formula $R^7$ represents the residue of a higher fatty acid containing 7 to 19 carbon atoms and $R^8$ represents a branched hydrocarbon chain containing 3 to 20 carbon atoms; hydrocarbons such as hexadecane, paraffin oil, perhydrosqualene; halogenated hydrocarbons such as perfluorodecahydronaphthalene; perfluorotributylamine; polysiloxanes; esters of organic acids, ethers and polyethers.

**16.** Composition according to one of Claims 13 to 15, characterized by the fact that the phase L contains at least one perfume and/or at least one fat-soluble active substance.

17. Composition according to one of Claims 1 to 16, characterized by the fact that the aqueous phase D contains at least one adjuvant chosen from the group consisting of opacifiers, gelling agents, flavourings, perfumes, sunscreens and colorants.

18. Composition according to Claim 17, characterized by the fact that the fat-soluble phase is chosen from the group consisting of a sunscreen, a substance intended to improve the condition of dry or aged skins or an anti-oxidant.

19. Food composition according to one of Claims 1 to 9, 13 and 14, characterized by the fact that the lipid(s) of formula (I) constituting the vesicle sheets is (are) chosen from edible lipids; that the aqueous phase E and the aqueous phase D optionally contain at least one water-soluble vitamin and that the constituent(s) of the phase L, when it is present, is (are) chosen from edible oils and optionally contain(s) at least one fat-soluble vitamin.

20. Process for the manufacture of a composition for cosmetic, pharmaceutical or food use, characterized by the fact that a dispersion in an aqueous medium D of lipid vesicles of lamellar structure encapsulating an aqueous phase E is prepared from a lipid phase which at least partially consists of at least one compound of formula I

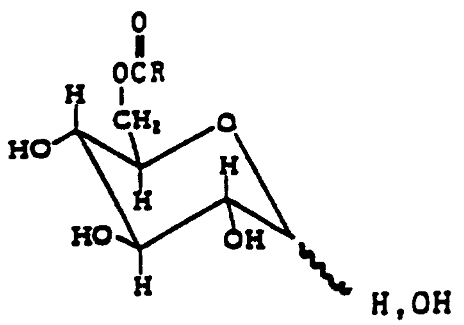

(I)

in which R represents a saturated or unsaturated linear hydrocarbon chain containing 9 to 17 carbon atoms.

**Patentansprüche**

**Patentansprüche für folgende Vertragsstaaten : AT, BE, CH, DE, DK, FR, GB, IT, LI, NL, SE**

1. Mittel zur kosmetischen, pharmazeutischen oder alimentären Anwendung, das in Dispersion in einem wäßrigen Milieu D Lipidvesikel mit lamellarer Struktur umfaßt, welche eine wäßrige Phase E einkapseln, dadurch gekennzeichnet, daß die Lipidphase, welche die Plättchen der erwähnten Vesikel bildet, wenigstens zum Teil aus einer Verbindung der Formel (I):

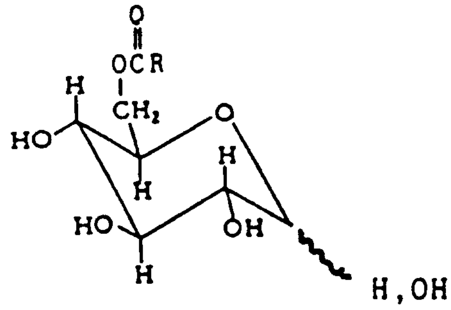

(I)

worin R eine lineare, gesättigte oder ungesättigte Kohlenwasserstoffkette mit 9 bis 17 Kohlenstoffatomen bedeutet, gebildet ist.

**2.** Mittel nach Anspruch 1, dadurch gekennzeichnet, daß die Verbindungen der Formel (I) ausgewählt sind unter denjenigen, bei denen der Acylrest R-CO- ein Decanoyl-, Dodecanoyl-, Myristoyl-, Hexadecanoyl-, Stearoyl-, Oleoyl-, Linoleoyl- oder Linolenoylrest ist.

**3.** Mittel nach Anspruch 2, dadurch gekennzeichnet, daß die Verbindungen der Formel (I) ausgewählt sind unter O-Oleoyl-6-D-glucose, O-Decanoyl-6-D-glucose, O-Dodecanoyl-6-D-glucose und O-Hexadecanoyl-6-D-glucose.

**4.** Mittel nach einem der Ansprüche 1 bis 3, dadurch gekennzeichnet, daß die Lipidphase, welche die Plättchen der Vesikel bildet, wenigstens ein zusätzliches ionisches und/oder nicht-ionisches amphiphiles Lipid natürlicher oder synthetischer Herkunft umfaßt, welches pro Molekül wenigstens eine hydrophile Gruppe aufweist, die ausgewählt ist unter einer Hydroxyl-, Etheroxid-, Carboxyl-, Phosphat- und Amingruppe.

**5.** Mittel nach Anspruch 4, dadurch gekennzeichnet, daß die zusätzlichen ionischen amphiphilen Lipide ausgewählt sind unter natürlichen oder synthetischen Phospholipiden, amphoteren Verbindungen und anionischen Verbindungen.

**6.** Mittel nach Anspruch 4, dadurch gekennzeichnet, daß die zusätzlichen nicht-ionischen amphiphilen Lipide ausgewählt sind unter:
(1) linearen oder verzweigten Polyglycerinethern der Formel:

$$R^0O - [C_3H_5(OH)O]_{\bar{n}} - H$$

worin:
. $-C_3H_5(OH)O$ für eine der folgenden Strukturen einzeln oder in Kombination steht:
$-CH_2CHOHCH_2O-$ ;

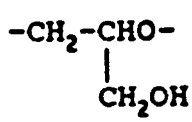 ; 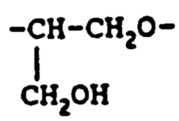 ;

. $\bar{n}$ einen statistischen Mittelwert zwischen 1 und 6 bedeutet;
. $R^0$ bedeutet:
(a) eine lineare oder verzweigte, gesättigte oder ungesättigte aliphatische Kette mit 12 bis 30 Kohlenstoffatomen; oder Kohlenwasserstoffreste von Lanolinalkoholen; oder Reste von langkettigen $\alpha$-Diolen;
(b) einen Rest $R^1CO$, worin $R^1$ für einen linearen oder verzweigten aliphatischen $C_{11}$-$C_{17}$-Rest steht;
(c) einen Rest $R^2[OC_2H_3(R^3)]$, worin:
. $R^2$ die für $R^0$ angegebenen Bedeutungen (a) oder (b) besitzen kann;
. $OC_2H_3(R^3)$- für eine der folgenden Strukturen einzeln oder in Kombination steht:

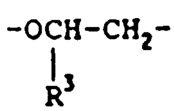 und 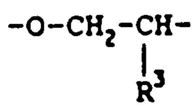

worin $R^3$ die für $R^0$ angegebene Bedeutung (a) besitzt;
(2) linearen oder verzweigten Polyglycerinethern, die zwei Fettketten aufweisen;
(3) polyoxyethylenierten Fettalkoholen und Sterolen und polyoxyethylenierten Phytosterolen;
(4) Polyolethern;
(5) gegebenenfalls oxyethyl nierten Polyolethern;
(6) Glycolipiden natürlicher oder synthetischer Herkunft;

(7) Hydroxyamiden der Formel:

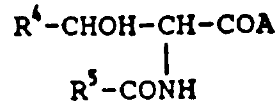

worin:

- $R^4$ einen $C_7$-$C_{21}$-Alkyl- oder -Alkenylrest bedeutet;
- $R^5$ einen gesättigten oder ungesättigten $C_7$-$C_{31}$-Kohlenwasserstoffrest bedeutet;
- COA eine Gruppe bedeutet, die ausgewählt ist unter den beiden folgenden Gruppen:
  . einem Rest

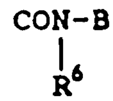

worin:

. B für einen von mono- oder polyhydroxylierten, primären oder sekundären Aminen abgeleiteten Rest steht; und

. $R^6$ ein Wasserstoffatom oder einen Methyl-, Ethyl- oder Hydroxyethylrest bedeutet; und

. einem Rest -COOZ, worin Z den Rest eines $C_3$-$C_7$-Polyolrestes bedeutet.

**7.** Mittel nach einem der Ansprüche 1 bis 6, dadurch gekennzeichnet, daß das die Vesikel bildende Lipid (die die Vesikel bildenden Lipide) mit wenigstens einem Additiv assoziiert ist(sind), das ausgewählt ist unter langkettigen Alkoholen und Diolen; Sterolen; langkettigen Aminen und ihren quaternären Ammoniumderivaten; Hydroxyalkylaminen, Dihydroxyalkylaminen, polyoxyethylenierten Fettaminen, Estern von Aminoalkoholen mit langer Kette und ihren Salzen und quaternären Ammoniumderivaten; Posphorsäureestern von Fettalkoholen; Alkylsulfaten; Polymeren vom Polypeptid- und Proteintyp; und Lipoprotiden, ausgewählt unter mono- oder polyacylierten Derivaten von Aminosäuren oder Polypeptiden, worin der Acylrest R'-CO- eine $C_{13}$-$C_{19}$-Kohlenwasserstoffkette R' umfaßt, wobei wenigstens eine der Funktionen, welche die Polypeptidkette oder den Aminosäurerest mit der lipophilen Kette verbindet, eine Amidfunktion ist, und wobei die Carboxylfunktionen der Polypeptidkette oder des Aminosäurerestes teilweise oder vollständig durch ein oder mehrere Alkalimetallkationen, ein Ammonium- oder ein von einem Amin abgeleitetes substituiertes Ammoniumion neutralisiert ist.

**8.** Mittel nach einem der Ansprüche 1 bis 7, dadurch gekennzeichnet, daß es 0,5 bis 25 Gew.-% des Lipids (der Lipide) enthält, welches (welche) die Plättchen der Vesikel bildet (bilden), wobei es sich bei diesen Angaben um Gewichtsprozent, bezogen auf das Gesamtgewicht des Mittels, handelt.

**9.** Mittel nach einem der Ansprüche 1 bis 8, dadurch gekennzeichnet, daß die wäßrige, in die Vesikel eingekapselte Phase E eine wäßrige Lösung der aktiven Substanz(en) ist.

**10.** Kosmetisches Mittel nach Anspruch 9, dadurch gekennzeichnet, daß die wäßrige Phase E und/oder die wäßrige Phase D wenigstens eine wasserlösliche kosmetische Substanz umfaßt (umfassen), ausgewählt unter Befeuchtungsmitteln, künstlichen Bräunungsmitteln, Mittel zur Färbung der Haut, Sonnenschutzmitteln, Sonnenfiltern, Antiperspirantien, Deodorantien, Adstringentien, erfrischenden Produkten, tonisierenden Produkten, narbenbildenden Produkten, keratolytischen Produkten, depilatorischen Produkten, Parfümwässern, wasserlöslichen Farbstoffen, Antischuppenmitteln, Antiseborrhöemitteln, Oxidationsmitteln, Reduktionsmitteln und Extrakten tierischer oder pflanzlicher Gewebe.

**11.** Pharmazeutisches Mittel nach Anspruch 9, dadurch gekennzeichnet, daß die wäßrige Phase E und/oder die wäßrige Phase D wenigstens ein Produkt umfaßt (umfassen), ausgewählt unter Vitaminen, Hormonen, Enzymen, Vaccinen, antiinflammatorischen, antibiotischen und bakteriziden Mitteln.

**12.** Mittel nach einem der Ansprüche 1 bis 11, dadurch gekennzeichnet, daß die Lipidphase, welche die Plättchen der Vesikel bildet, wenigstens eine fettlösliche aktive Substanz enthält.

**13.** Mittel nach Anspruch 12, dadurch gekennzeichnet, daß die fettlösliche aktive Substanz ausgewählt ist unter fettlöslichen Sonnenfiltern, Substanzen zur Verbesserung des Zustandes trockener oder seniler Haut, Tocopherolen, den Vitaminen E oder F, Vitamin A und den Estern davon, Retinoesäure, Antioxidantien, essentiellen Fettsäuren, Glycyrrhetinsäure, keratolytischen Mitteln und Carotinoiden.

**14.** Mittel nach einem der Ansprüche 1 bis 13, dadurch gekennzeichnet, daß wenigstens eine mit Wasser nicht mischbare flüssige Phase L in der wäßrigen Phase D dispergiert ist.

**15.** Mittel nach Anspruch 14, dadurch gekennzeichnet, daS es 2 bis 70 Gew.-%, bezogen auf das Gesamtgewicht des Mittels, der mit Wasser nicht mischbaren flüssigen Phase L enthält, wobei das Gewichtsverhältnis von dem die Kügelchen bildenden Lipid (den die Kügelchen bildenden Lipiden) zu der dispergierten flüssigen Phase L im Bereich von 0,02/1 bis 10/1 liegt.

**16.** Mittel nach einem der Ansprüche 14 oder 15, dadurch gekennzeichnet, daß der (die) die flüssige Phase L, die in der wäßrigen Phase D dispergiert ist, bildende Bestandteil (bildenden Bestandteile) ausgewählt ist (sind) unter Ölen, wie Estern von Fettsäuren und Polyolen und Estern von Fettsäuren und verzweigten Alkoholen der Formel $R^7$-$COOR^8$, worin $R^7$ für einen höheren Fettsäurerest mit 7 bis 19 Kohlenstoffatomen steht und $R^8$ für eine verzweigte Kohlenwasserstoffkette mit 3 bis 20 Kohlenstoffatomen steht; Kohlenwasserstoffen, wie Hexadecan, Paraffinöl, Perhydrosqualen; Halogenkohlenstoffen, wie Perfluorodecahydronaphthalin; Perfluorotributylamin; Polysiloxanen; Estern organischer Säuren, Ethern und Polyethern.

**17.** Mittel nach einem der Ansprüche 14 bis 16, dadurch gekennzeichnet, daS die Phase L wenigstens ein Parfüm und/oder wenigstens eine fettlösliche aktive Substanz umfaßt.

**18.** Mittel nach einem der Ansprüche 1 bis 17, dadurch gekennzeichnet, daß die wäßrige Phase D wenigstens ein Adjuvans umfaßt, das ausgewählt ist unter opakmachenden Mitteln, gelbildenden Mitteln, Aromastoffen, Parfüms, Antisonnenfiltern und Farbstoffen.

**19.** Mittel nach Anspruch 18, dadurch gekennzeichnet, daß die fettlösliche Phase ausgewählt ist unter einem Antisonnenfilter, einer Substanz zur Verbesserung des Zustandes trockener oder seniler Haut oder einem Antioxidans.

**20.** Alimentäres Mittel nach einem der Ansprüche 1 bis 9, 14 und 15, dadurch gekennzeichnet, daß das Lipid (die Lipide) der Formel (I), welches (welche) die Plättchen der Vesikel bildet (bilden), ausgewählt ist (sind) unter genießbaren Lipiden; daß die wäßrige Phase E und die wäßrige Phase D gegebenenfalls wenigstens ein wasserlösliches Vitamin umfassen und daß der (die) die Phase L bildende Bestandteil (bildenden Bestandteile), wenn diese vorhanden ist, ausgewählt ist (sind) unter genießbaren Ölen und gegebenenfalls wenigstens ein fettlösliches Vitamin umfaßt (umfassen).

**Patentansprüche für folgenden Vertragsstaat : ES**

**1.** Mittel zur kosmetischen Anwendung, das in Dispersion in einem wäßrigen Milieu D Lipidvesikel mit lamellarer Struktur umfaßt, welche eine wäßrige Phase E einkapseln, dadurch gekennzeichnet, daß die Lipidphase, welche die Plättchen der erwähnten Vesikel bildet, wenigstens zum Teil aus einer Verbindung der Formel (I):

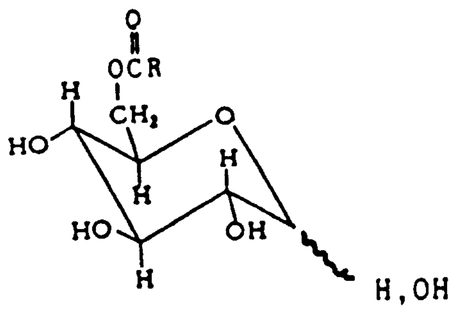

(I)

worin R eine lineare, gesättigte oder ungesättigte Kohlenwasserstoffkette mit 9 bis 17 Kohlenstoffatomen bedeutet, gebildet ist.

2. Mittel nach Anspruch 1, dadurch gekennzeichnet, daß die Verbindungen der Formel (I) ausgewählt sind unter denjenigen, bei denen der Acylrest R-CO- ein Decanoyl-, Dodecanoyl-, Myristoyl-, Hexadecanoyl-, Stearoyl-, Oleoyl-, Linoleoyl- oder Linolenoylrest ist.

3. Mittel nach Anspruch 2, dadurch gekennzeichnet, daß die Verbindungen der Formel (I) ausgewählt sind unter O-Oleoyl-6-D-glucose, O-Decanoyl-6-D-glucose, O-Dodecanoyl-6-D-glucose und O-Hexadecanoyl-6-D-glucose.

4. Mittel nach einem der Ansprüche 1 bis 3, dadurch gekennzeichnet, daß die Lipidphase, welche die Plättchen der Vesikel bildet, wenigstens ein zusätzliches ionisches und/oder nicht-ionisches amphiphiles Lipid natürlicher oder synthetischer Herkunft umfaßt, welches pro Molekül wenigstens eine hydrophile Gruppe aufweist, die ausgewählt ist unter einer Hydroxyl-, Etheroxid-, Carboxyl-, Phosphat- und Amingruppe.

5. Mittel nach Anspruch 4, dadurch gekennzeichnet, daß die zusätzlichen ionischen amphiphilen Lipide ausgewählt sind unter natürlichen oder synthetischen Phospholipiden, amphoteren Verbindungen und anionischen Verbindungen.

6. Mittel nach Anspruch 4, dadurch gekennzeichnet, daß die zusätzlichen nicht-ionischen amphiphilen Lipide ausgewählt sind unter:
(1) linearen oder verzweigten Polyglycerinethern der Formel:

$$R^{o}O - [C_3H_5(OH)O]_{\bar{n}} - H$$

worin:
. $-C_3H_5(OH)O$ für eine der folgenden Strukturen einzeln oder in Kombination steht:
$-CH_2CHOHCH_2O-$ ;

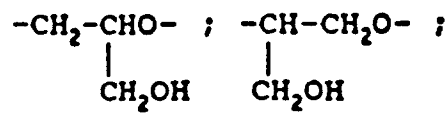

. $\bar{n}$ einen statistischen Mittelwert zwischen 1 und 6 bedeutet;
. $R^0$ bedeutet:
(a) eine lineare oder verzweigte, gesättigte oder ungesättigte aliphatische Kette mit 12 bis 30 Kohlenstoffatomen; oder Kohlenwasserstoffreste von Lanolinalkoholen; oder Reste von langkettigen $\alpha$-Diolen;

(b) einen Rest $R^1CO$, worin $R^1$ für einen linearen oder verzweigten aliphatischen $C_{11}$-$C_{17}$-Rest steht;

(c) einen Rest $R^2\{OC_2H_3(R^3)\}$, worin:

. $R^2$ die für $R^0$ angegebenen Bedeutungen (a) oder (b) besitzen kann;

. $OC_2H_3(R^3)$- für eine der folgenden Strukturen einzeln oder in Kombination steht:

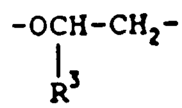 und 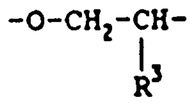

worin $R^3$ die für $R^0$ angegebene Bedeutung (a) besitzt;

(2) linearen oder verzweigten Polyglycerinethern, die zwei Fettketten aufweisen;

(3) polyoxyethylenierten Fettalkoholen und Sterolen und polyoxyethylenierten Phytosterolen;

(4) Polyolethern;

(5) gegebenenfalls oxyethylenierten Polyolethern;

(6) Glycolipiden natürlicher oder synthetischer Herkunft;

(7) Hydroxyamiden der Formel:

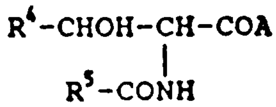

worin:

- $R^4$ einen $C_7$-$C_{21}$-Alkyl- oder -Alkenylrest bedeutet;
- $R^5$ einen gesättigten oder ungesättigten $C_7$-$C_{31}$-Kohlenwasserstoffrest bedeutet;
- COA eine Gruppe bedeutet, die ausgewählt ist unter den beiden folgenden Gruppen:

. einem Rest

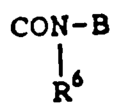

worin:

. B für einen von mono- oder polyhydroxylierten, primären oder sekundären Aminen abgeleiteten Rest steht; und

. $R^6$ ein Wasserstoffatom oder einen Methyl-, Ethyl- oder Hydroxyethylrest bedeutet; und

. einem Rest -COOZ, worin Z den Rest eines $C_3$-$C_7$-Polyolrestes bedeutet.

**7.** Mittel nach einem der Ansprüche 1 bis 6, dadurch gekennzeichnet, daß das die Vesikel bildende Lipid (die die Vesikel bildenden Lipide) mit wenigstens einem Additiv assoziiert ist(sind), das ausgewählt ist unter langkettigen Alkoholen und Diolen; Sterolen; langkettigen Aminen und ihren quaternären Ammoniumderivaten; Hydroxyalkylaminen, Dihydroxyalkylaminen, polyoxyethylenierten Fettaminen, Estern von Aminoalkoholen mit langer Kette und ihren Salzen und quaternären Ammoniumderivaten; Posphorsäureestern von Fettalkoholen; Alkylsulfaten; Polymeren vom Polypeptid- und Proteintyp; und Lipoprotiden, ausgewählt unter mono- oder polyacylierten Derivaten von Aminosäuren oder Polypeptiden, worin der Acylrest R'-CO- eine $C_{13}$-$C_{19}$-Kohlenwasserstoffkette R' umfaßt, wobei wenigstens eine der Funktionen, welche die Polypeptidkette oder den Aminosäurerest mit der lipophilen Kette verbindet, eine Amidfunktion ist, und wobei die Carboxylfunktionen der Polypeptidkette oder des Aminosäurerestes teilweise oder vollständig durch ein oder mehrere Alkalimetallkationen, ein Ammonium- oder ein von einem Amin abgeleitetes substituiertes Ammoniumion neutralisiert ist.

**8.** Mittel nach einem der Ansprüche 1 bis 7, dadurch gekennzeichnet, daß es 0,5 bis 25 Gew.-% des Lipids (der Lipide) enthält, welches (welche) die Plättchen der Vesikel bildet (bilden), wobei es sich bei

diesen Angaben um Gewichtsprozent, bezogen auf das Gesamtgewicht des Mittels, handelt.

**9.** Mittel nach einem der Ansprüche 1 bis 8, dadurch gekennzeichnet, daß die wäßrige, in die Vesikel eingekapselte Phase E eine wäßrige Lösung der aktiven Substanz(en) ist.

**10.** Kosmetisches Mittel nach Anspruch 9, dadurch gekennzeichnet, daß die wäßrige Phase E und/oder die wäßrige Phase D wenigstens eine wasserlösliche kosmetische Substanz umfaßt (umfassen), ausgewählt unter Befeuchtungsmitteln, künstlichen Bräunungsmitteln, Mittel zur Färbung der Haut, Sonnenschutzmitteln, Sonnenfiltern, Antiperspirantien, Deodorantien, Adstringentien, erfrischenden Produkten, tonisierenden Produkten, narbenbildenden Produkten, keratolytischen Produkten, depilatorischen Produkten, Parfümwässern, wasserlöslichen Farbstoffen, Antischuppenmitteln, Antiseborrhöemitteln, Oxidationsmitteln, Reduktionsmitteln und Extrakten tierischer oder pflanzlicher Gewebe.

**11.** Mittel nach einem der Ansprüche 1 bis 10, dadurch gekennzeichnet, daß die Lipidphase, welche die Plättchen der Vesikel bildet, wenigstens eine fettlösliche aktive Substanz enthält.

**12.** Mittel nach Anspruch 11, dadurch gekennzeichnet, daß die fettlösliche aktive Substanz ausgewählt ist unter fettlöslichen Sonnenfiltern, Substanzen zur Verbesserung des Zustandes trockener oder seniler Haut, Tocopherolen, den Vitaminen E oder F, Vitamin A und den Estern davon, Retinoesäure, Antioxidantien, essentiellen Fettsäuren, Glycyrrhetinsäure, keratolytischen Mitteln und Carotinoiden.

**13.** Mittel nach einem der Ansprüche 1 bis 12, dadurch gekennzeichnet, daß wenigstens eine mit Wasser nicht mischbare flüssige Phase L in der wäßrigen Phase D dispergiert ist.

**14.** Mittel nach Anspruch 13, dadurch gekennzeichnet, daß es 2 bis 70 Gew.-%, bezogen auf das Gesamtgewicht des Mittels, der mit Wasser nicht mischbaren flüssigen Phase L enthält, wobei das Gewichtsverhältnis von dem die Kügelchen bildenden Lipid (den die Kügelchen bildenden Lipiden) zu der dispergierten flüssigen Phase L im Bereich von 0,02/1 bis 10/1 liegt.

**15.** Mittel nach einem der Ansprüche 13 oder 14, dadurch gekennzeichnet, daß der (die) die flüssige Phase L, die in der wäßrigen Phase D dispergiert ist, bildende Bestandteil (bildenden Bestandteile) ausgewählt ist (sind) unter Ölen, wie Estern von Fettsäuren und Polyolen und Estern von Fettsäuren und verzweigten Alkoholen der Formel $R^7$-$COOR^8$, worin $R^7$ für einen höheren Fettsäurerest mit 7 bis 19 Kohlenstoffatomen steht und $R^8$ für eine verzweigte Kohlenwasserstoffkette mit 3 bis 20 Kohlenstoffatomen steht; Kohlenwasserstoffen, wie Hexadecan, Paraffinöl, Perhydrosqualen; Halogenkohlenstoffen, wie Perfluorodecahydronaphthalin; Perfluorotributylamin; Polysiloxanen; Estern organischer Säuren, Ethern und Polyethern.

**16.** Mittel nach einem der Ansprüche 13 bis 15, dadurch gekennzeichnet, daß die Phase L wenigstens ein Parfüm und/oder wenigstens eine fettlösliche aktive Substanz umfaßt.

**17.** Mittel nach einem der Ansprüche 1 bis 16, dadurch gekennzeichnet, daß die wäßrige Phase D wenigstens ein Adjuvans umfaßt, das ausgewählt ist unter opakmachenden Mitteln, gelbildenden Mitteln, Aromastoffen, Parfüms, Antisonnenfiltern und Farbstoffen.

**18.** Mittel nach Anspruch 17, dadurch gekennzeichnet, daß die fettlösliche Phase ausgewählt ist unter einem Antisonnenfilter, einer Substanz zur Verbesserung des Zustandes trockener oder seniler Haut oder einem Antioxidans.

**19.** Verfahren zur Herstellung eines Mittels zur kosmetischen, pharmazeutischen oder alimentären Anwendung, dadurch gekennzeichnet, daS man eine Dispersion von Lipidvesikel mit lamellarer Struktur, welche eine wäßrige Phase E einkapseln, in einem wäßrigen Milieu D ausgehend von einer Lipidphase herstellt, welche wenigstens zum Teil aus einer Verbindung der Formel (I):

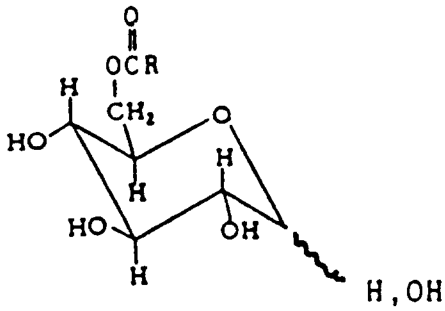

(I)

worin R eine lineare, gesättigte oder ungesättigte Kohlenwasserstoffkette mit 9 bis 17 Kohlenstoffatomen bedeutet, gebildet ist.

**Patentansprüche für folgenden Vertragsstaat : GR**

1. Mittel zur kosmetischen oder alimentären Anwendung, das in Dispersion in einem wäßrigen Milieu D Lipidvesikel mit lamellarer Struktur umfaßt, welche eine wäßrige Phase E einkapseln, dadurch gekennzeichnet, daß die Lipidphase, welche die Plättchen der erwähnten Vesikel bildet, wenigstens zum Teil aus einer Verbindung der Formel (I):

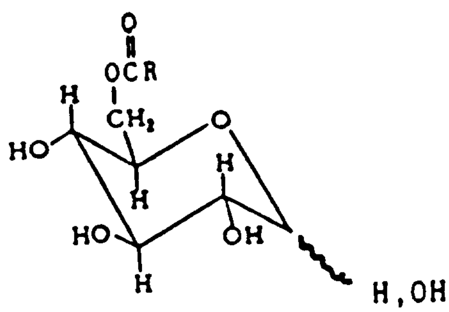

(I)

worin R eine lineare, gesättigte oder ungesättigte Kohlenwasserstoffkette mit 9 bis 17 Kohlenstoffatomen bedeutet, gebildet ist.

2. Mittel nach Anspruch 1, dadurch gekennzeichnet, daß die Verbindungen der Formel (I) ausgewählt sind unter denjenigen, bei denen der Acylrest R-CO- ein Decanoyl-, Dodecanoyl-, Myristoyl-, Hexadecanoyl-, Stearoyl-, Oleoyl-, Linoleoyl- oder Linolenoylrest ist.

3. Mittel nach Anspruch 2, dadurch gekennzeichnet, daß die Verbindungen der Formel (I) ausgewählt sind unter O-Oleoyl-6-D-glucose, O-Decanoyl-6-D-glucose, O-Dodecanoyl-6-D-glucose und O-Hexadecanoyl-6-D-glucose.

4. Mittel nach einem der Ansprüche 1 bis 3, dadurch gekennzeichnet, daß die Lipidphase, welche die Plättchen der Vesikel bildet, wenigstens ein zusätzliches ionisches und/oder nicht-ionisches amphiphiles Lipid natürlicher oder synthetischer Herkunft umfaßt, welches pro Molekül wenigstens eine hydrophile Gruppe aufweist, die ausgewählt ist unter einer Hydroxyl-, Etheroxid-, Carboxyl-, Phosphat- und Amingruppe.

5. Mittel nach Anspruch 4, dadurch gekennzeichnet, daß die zusätzlichen ionischen amphiphilen Lipide ausgewählt sind unter natürlichen oder synthetischen Phospholipiden, amphoteren Verbindungen und anionischen Verbindungen.

6. Mittel nach Anspruch 4, dadurch gekennzeichnet, daß die zusätzlichen nicht-ionischen amphiphilen Lipide ausgewählt sind unter:

(1) linearen oder verzweigten Polyglycerinethern der Formel:

$$R^0O-[C_3H_5(OH)O]_{\bar{n}}-H$$

worin:

. $-C_3H_5(OH)O$ für eine der folgenden Strukturen einzeln oder in Kombination steht:
$-CH_2CHOHCH_2O-$ ;

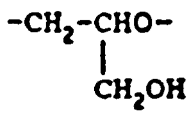 ; 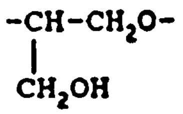 ;

. $\bar{n}$ einen statistischen Mittelwert zwischen 1 und 6 bedeutet;

. $R^0$ bedeutet:

(a) eine lineare oder verzweigte, gesättigte oder ungesättigte aliphatische Kette mit 12 bis 30 Kohlenstoffatomen; oder Kohlenwasserstoffreste von Lanolinalkoholen; oder Reste von langkettigen $\alpha$-Diolen;

(b) einen Rest $R^1CO$, worin $R^1$ für einen linearen oder verzweigten aliphatischen $C_{11}$-$C_{17}$-Rest steht;

(c) einen Rest $R^2[OC_2H_3(R^3)]$, worin:

. $R^2$ die für $R^0$ angegebenen Bedeutungen (a) oder (b) besitzen kann;

. $OC_2H_3(R^3)$- für eine der folgenden Strukturen einzeln oder in Kombination steht:

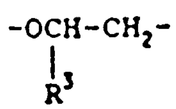 und 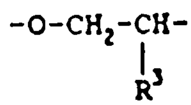

worin $R^3$ die für $R^0$ angegebene Bedeutung (a) besitzt;

(2) linearen oder verzweigten Polyglycerinethern, die zwei Fettketten aufweisen;

(3) polyoxyethylenierten Fettalkoholen und Sterolen und polyoxyethylenierten Phytosterolen;

(4) Polyolethern;

(5) gegebenenfalls oxyethyl nierten Polyolethern;

(6) Glycolipiden natürlicher oder synthetischer Herkunft;

(7) Hydroxyamiden der Formel:

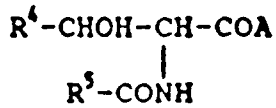

worin:

- $R^4$ einen $C_7$-$C_{21}$-Alkyl- oder -Alkenylrest bedeutet;
- $R^5$ einen gesättigten oder ungesättigten $C_7$-$C_{31}$-Kohlenwasserstoffrest bedeutet;
- COA eine Gruppe bedeutet, die ausgewählt ist unter den beiden folgenden Gruppen:
  . einem Rest

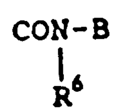

worin:

. B für einen von mono- oder polyhydroxylierten, primären oder sekundären Aminen abgeleiteten Rest steht; und

. $R^6$ ein Wasserstoffatom oder einen Methyl-, Ethyl- oder Hydroxyethylrest bedeutet; und

. einem Rest -COOZ, worin Z den Rest eines $C_3$-$C_7$-Polyolrestes bedeutet.

**7.** Mittel nach einem der Ansprüche 1 bis 6, dadurch gekennzeichnet, daß das die Vesikel bildende Lipid (die die Vesikel bildenden Lipide) mit wenigstens einem Additiv assoziiert ist(sind), das ausgewählt ist unter langkettigen Alkoholen und Diolen; Sterolen; langkettigen Aminen und ihren quaternären Ammoniumderivaten; Hydroxyalkylaminen,, Dihydroxyalkylaminen, polyoxyethylenierten Fettaminen, Estern von Aminoalkoholen mit langer Kette und ihren Salzen und quaternären Ammoniumderivaten; Posphorsäureestern von Fettalkoholen; Alkylsulfaten; Polymeren vom Polypeptid- und Proteintyp; und Lipoprotiden, ausgewählt unter mono- oder polyacylierten Derivaten von Aminosäuren oder Polypeptiden, worin der Acylrest R'-CO- eine $C_{13}$-$C_{19}$-Kohlenwasserstoffkette R' umfaßt, wobei wenigstens eine der Funktionen, welche die Polypeptidkette oder den Aminosäurerest mit der lipophilen Kette verbindet, eine Amidfunktion ist, und wobei die Carboxylfunktionen der Polypeptidkette oder des Aminosäurerestes teilweise oder vollständig durch ein oder mehrere Alkalimetallkationen, ein Ammonium- oder ein von einem Amin abgeleitetes substituiertes Ammoniumion neutralisiert ist.

**8.** Mittel nach einem der Ansprüche 1 bis 7, dadurch gekennzeichnet, daß es 0,5 bis 25 Gew.-% des Lipids (der Lipide) enthält, welches (welche) die Plättchen der Vesikel bildet (bilden), wobei es sich bei diesen Angaben um Gewichtsprozent, bezogen auf das Gesamtgewicht des Mittels, handelt.

**9.** Mittel nach einem der Ansprüche 1 bis 8, dadurch gekennzeichnet, daß die wäßrige, in die Vesikel eingekapselte Phase E eine wäßrige Lösung der aktiven Substanz(en) ist.

**10.** Kosmetisches Mittel nach Anspruch 9, dadurch gekennzeichnet, daß die wäßrige Phase E und/oder die wäßrige Phase D wenigstens eine wasserlösliche kosmetische Substanz umfaßt (umfassen), ausgewählt unter Befeuchtungsmitteln, künstlichen Bräunungsmitteln, Mittel zur Färbung der Haut, Sonnenschutzmitteln, Sonnenfiltern, Antiperspirantien, Deodorantien, Adstringentien, erfrischenden Produkten, tonisierenden Produkten, narbenbildenden Produkten, keratolytischen Produkten, depilatorischen Produkten, Parfümwässern, wasserlöslichen Farbstoffen, Antischuppenmitteln, Antiseborrhöemitteln, Oxidationsmitteln, Reduktionsmitteln und Extrakten tierischer oder pflanzlicher Gewebe.

**11.** Mittel nach einem der Ansprüche 1 bis 10, dadurch gekennzeichnet, daß die Lipidphase, welche die Plättchen der Vesikel bildet, wenigstens eine fettlösliche aktive Substanz enthält.

**12.** Mittel nach Anspruch 11, dadurch gekennzeichnet, daß die fettlösliche aktive Substanz ausgewählt ist unter fettlöslichen sonnenfiltern, Substanzen zur Verbesserung des Zustandes trockener oder seniler Haut, Tocopherolen, den Vitaminen E oder F, Vitamin A und den Estern davon, Retinoesäure, Antioxidantien, essentiellen Fettsäuren, Glycyrrhetinsäure, keratolytischen Mitteln und Carotinoiden.

**13.** Mittel nach einem der Ansprüche 1 bis 12, dadurch gekennzeichnet, daß wenigstens eine mit Wasser nicht mischbare flüssige Phase L in der wäßrigen Phase D dispergiert ist.

**14.** Mittel nach Anspruch 13, dadurch gekennzeichnet, daß es 2 bis 70 Gew.-%, bezogen auf das Gesamtgewicht des Mittels, der mit Wasser nicht mischbaren flüssigen Phase L enthält, wobei das Gewichtsverhältnis von dem die Kügelchen bildenden Lipid (den die Kügelchen bildenden Lipiden) zu der dispergierten flüssigen Phase L im Bereich von 0,02/1 bis 10/1 liegt.

**15.** Mittel nach einem der Ansprüche 13 oder 14, dadurch gekennzeichnet, daß der (die) die flüssige Phase L, die in der wäßrigen Phase D dispergiert ist, bildende Bestandteil (bildenden Bestandteile) ausgewählt ist (sind) unter Ölen, wie Estern von Fettsäuren und Polyolen und Estern von Fettsäuren und verzweigten Alkoholen der Formel $R^7$-$COOR^8$, worin $R^7$ für einen höheren Fettsäurerest mit 7 bis 19 Kohlenstoffatomen steht und $R^8$ für eine verzweigte Kohlenwasserstoffkette mit 3 bis 20 Kohlenstoffatomen steht; Kohlenwasserstoffen, wie Hexadecan, Paraffinöl, Perhydrosqualen; Halogenkohlenstoffen, wie Perfluorodecahydronaphthalin; Perfluorotributylamin; Polysiloxanen; Estern organischer Säuren, Ethern und Polyethern.

**16.** Mittel nach einem der Ansprüche 13 bis 15, dadurch gekennzeichnet, daß die Phase L wenigstens ein Parfüm und/oder wenigstens eine fettlösliche aktive Substanz umfaßt.

**17.** Mittel nach einem der Ansprüche 1 bis 16, dadurch gekennzeichnet, daß die wäßrige Phase D wenigstens ein Adjuvans umfaßt, das ausgewählt ist unter opakmachenden Mitteln, gelbildenden Mitteln, Aromastoffen, Parfüms, Antisonnenfiltern und Farbstoffen.

**18.** Mittel nach Anspruch 17, dadurch gekennzeichnet, daß die fettlösliche Phase ausgewählt ist unter einem Antisonnenfilter, einer Substanz zur Verbesserung des Zustandes trockener oder seniler Haut oder einem Antioxidans.

**19.** Alimentäres Mittel nach einem der Ansprüche 1 bis 9, 13 und 14, dadurch gekennzeichnet, daß das Lipid (die Lipide) der Formel (I), welches (welche) die Plättchen der Vesikel bildet (bilden), ausgewählt ist (sind) unter genießbaren Lipiden; daß die wäßrige Phase E und die wäßrige Phase D gegebenenfalls wenigstens ein wasserlösliches Vitamin umfassen und daß der (die) die Phase L bildende Bestandteil (bildenden Bestandteile), wenn diese vorhanden ist, ausgewählt ist (sind) unter genießbaren Ölen und gegebenenfalls wenigstens ein fettlösliches Vitamin umfaßt (umfassen).

**20.** Verfahren zur Herstellung eines Mittels zur kosmetischen, pharmazeutischen oder alimentären Anwendung, dadurch gekennzeichnet, daß man eine Dispersion von Lipidvesikel mit lamellarer Struktur, welche eine wäßrige Phase E einkapseln, in einem wäßrigen Milieu D ausgehend von einer Lipidphase herstellt, welche wenigstens zum Teil aus einer Verbindung der Formel (I):

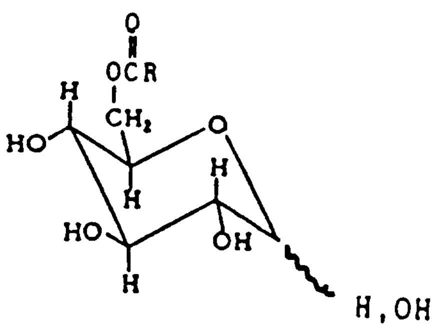

(I)

worin R eine lineare, gesättigte oder ungesättigte Kohlenwasserstoffkette mit 9 bis 17 Kohlenstoffatomen bedeutet, gebildet ist.